(19) Europäisches Patentamt
European Patent Office
Office européen des brevets

(11) EP 4 740 965 A1

(12) **EUROPEAN PATENT APPLICATION**
published in accordance with Art. 153(4) EPC

(43) Date of publication:
**13.05.2026 Bulletin 2026/20**

(21) Application number: **24835385.6**

(22) Date of filing: **04.07.2024**

(51) International Patent Classification (IPC):
***A61K 47/64*** (2017.01) ***A61K 31/519*** (2006.01)
***A61K 9/20*** (2006.01) ***A61K 9/08*** (2006.01)
***A61K 9/14*** (2006.01) ***A61P 35/00*** (2006.01)
***A61P 37/06*** (2006.01) ***A61P 37/02*** (2006.01)

(52) Cooperative Patent Classification (CPC):
**A61K 9/08; A61K 9/14; A61K 9/20; A61K 31/519; A61K 47/64; A61P 35/00; A61P 37/02; A61P 37/06**

(86) International application number:
**PCT/CN2024/103473**

(87) International publication number:
**WO 2025/007905 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.07.2023 CN 202310811322**

(71) Applicant: **Xizang Haisco Pharmaceutical Co., Ltd.**
**Lhoka, Tibet 856099 (CN)**

(72) Inventors:
- **LI, Xiaoping**
  **Chengdu, Sichuan 611130 (CN)**
- **GUAN, Lin**
  **Chengdu, Sichuan 611130 (CN)**
- **XU, Zaiyang**
  **Chengdu, Sichuan 611130 (CN)**
- **BI, Xianshuang**
  **Chengdu, Sichuan 611130 (CN)**

(74) Representative: **AWA Denmark A/S**
**Strandgade 56**
**1401 Copenhagen K (DK)**

# (54) PHARMACEUTICAL COMPOSITION OF COMPOUND FOR INHIBITING AND DEGRADING IRAK4, AND USE THEREOF IN MEDICINE

(57) A pharmaceutical composition of a compound for inhibiting and degrading IRAK4, and the use thereof in medicine. The pharmaceutical composition comprises an active ingredient A and a pharmaceutically acceptable excipient, wherein the active ingredient A is selected from the compound as represented by general formula (I) or a stereoisomer, a tautomer, a deuterated substance, a solvate, a prodrug, a metabolite, a pharmaceutically acceptable salt or an eutectic crystal thereof. The pharmaceutical composition comprises 0.5-800 mg of the active ingredient A, and the content of the active ingredient A is selected from 0.5%-99.0%. The present invention also relates to the use of the pharmaceutical composition in the preparation of a relevant drug for treating tumours or autoimmune system diseases. B-L-K (I).

EP 4 740 965 A1

## Description

### Technical Field

The present invention belongs to the field of pharmaceutical formulations, and in particular relates to a pharmaceutical composition, which comprises a therapeutically effective amount of an active ingredient A and a pharmaceutically acceptable excipient, wherein the active ingredient A is selected from a compound as represented by general formula (I) or a stereoisomer, a tautomer, a deuterated substance, a solvate, a prodrug, a metabolite, a pharmaceutically acceptable salt or an eutectic crystal thereof; and the pharmaceutical composition comprises 0.5-800 mg of the active ingredient A, and the content of the active ingredient A is selected from 0.5%-99.0%. The present invention also relates to the use of the pharmaceutical composition in the preparation of a relevant drug for treating tumours or autoimmune system diseases.

### Background Art

Kinases catalyse the phosphorylation of proteins, lipids, sugars, nucleosides, and other cellular metabolites and play key roles in various aspects of physiology in eukaryotic cells. In particular, protein kinases and lipid kinases are involved in controlling the activated signal events in cells for growth, differentiation and survival in response to extracellular mediators or stimuli such as growth factors, cytokines or chemokines. In general, protein kinases are divided into two classes, one that preferentially phosphorylates tyrosine residues and the other that preferentially phosphorylates serine and/or threonine residues.

Interleukin-1 receptor kinase 4 (IRAK4) is a serine/threonine-specific protein kinase that belongs to the tyrosine-like kinase (TLK) family and is a key factor in the innate immune response involving interleukin-1, interleukin-18, and interleukin-33 receptors and Toll-like receptors. After extracellular signal molecules bind to interleukin receptors or Toll-like receptors, they are recruited to form a MyD88:IRAK4:IRAKl/2 multiprotein complex, leading to the phosphorylation of IRAK1/2 and mediating a series of downstream signallings, thereby activating the p38, JNK and NF-kB signalling pathways, and ultimately leading to the expression of pro-inflammatory cytokines. Clinical pathological studies have shown that individuals with IRAK4 mutations are protected against chronic lung disease and inflammatory bowel disease. IRAK4 deficiency itself is not lethal; individuals survive to adulthood and their risk of infection decreases with age. Therefore, IRAK4 has become an important therapeutic target and has attracted widespread research and development interest.

PROTAC (proteolysis targeting chimera) molecules are a class of bifunctional compounds that can simultaneously bind targeting proteins and E3 ubiquitin ligases. Such compounds can be recognized by proteasomes of cells, causing the degradation of targeting proteins, and can effectively reduce the content of targeting proteins in the cells. By introducing a ligand capable of binding to various targeting proteins into the PROTAC molecules, it is possible to apply the PROTAC technology to the treatment of various diseases, and this technology has attracted extensive attention in recent years.

Therefore, it is necessary to develop a novel IRAK4 inhibitor and a PROTAC drug of E3 ubiquitin ligase for the treatment of IRAK4-related diseases.

The patent PCT/CN2023/070367 recites compounds 19, 20, 24, and 25, which have good IRAK4 inhibitory or degrading activity. When the compound is used as a therapeutic agent for treating humans, the administration of a therapeutically effective dose becomes problematic and may lead to toxic side effects or ineffective therapy. Therefore, it is essential to develop a pharmaceutical composition or a pharmaceutical formulation with good safety, effectiveness and stability.

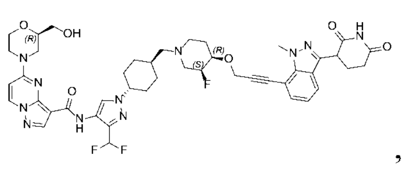
,
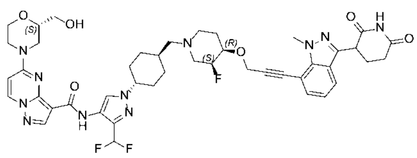
,

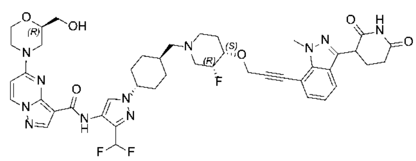
,
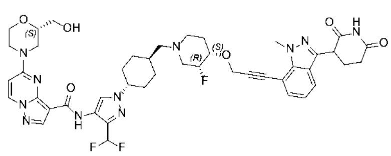
.

Summary of the Invention

The objective of the present invention relates to a pharmaceutical composition, which comprises an active ingredient A and a pharmaceutically acceptable excipient, wherein the active ingredient A is selected from a compound as represented by general formula (I) or a stereoisomer, a tautomer, a deuterated substance, a solvate, a prodrug, a metabolite, a pharmaceutically acceptable salt or an eutectic crystal thereof. The present invention also relates to the use of the pharmaceutical composition in the preparation of a relevant drug for treating tumours or autoimmune system diseases.

The pharmaceutical composition of the present invention has stable quality, a high dissolution rate, good absorption, low irritation, and good safety, and is orally absorbable.

The present invention relates to a pharmaceutical composition, which comprises a therapeutically effective amount of an active ingredient A and a pharmaceutically acceptable excipient. The pharmaceutical composition can be in a unit formulation form.

The present invention relates to a pharmaceutical composition, which comprises an active ingredient A and a pharmaceutically acceptable excipient, wherein the active ingredient A is selected from a compound as represented by general formula (I) or a stereoisomer, a tautomer, a deuterated substance, a solvate, a prodrug, a metabolite, a pharmaceutically acceptable salt or an eutectic crystal thereof,

B-L-K (I);

in certain embodiments, L is -Ak1-Cy1-Ak2-Cy2-Ak3-Cy3-Ak4-Cy4-Ak5-;

Ak1, Ak2, Ak3, Ak4 and Ak5 are each independently selected from $-(CH_2)_q-$, O, $-(CH_2)_qNR^L-$, $NR^LC=O$, $C=ONR^L$, C=O, $-R^LC=CR^L-$, C≡C or a bond;

$R^L$ is selected from H or $C_{1-6}$ alkyl;

Cy1, Cy2, Cy3 and Cy4 are each independently selected from a bond, 4- to 7-membered mono-heterocyclic ring, 4- to 10-membered fused-heterocyclic ring, 5-to 12-membered spiro-heterocyclic ring, 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S, or N;

B is selected from

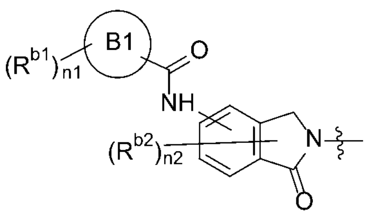

or

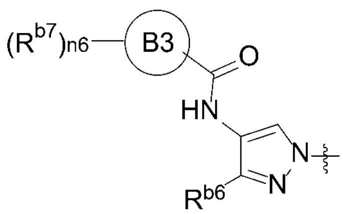

;

B1 and B3 are each independently selected from $C_{6-10}$ aryl, 5- to 10-membered heteroaryl or 4- to 10-membered heterocyclyl, wherein the heteroaryl or heterocyclyl contains 1 to 4 heteroatoms selected from O, S or N;

$R^{b1}$ and $R^{b7}$ are each independently selected from H, F, Cl, Br, **I,** =O, OH, $NH_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $-N(R^{b21})_2$, $C_{6-10}$ aryl, 5- to 10-membered heteroaryl or 4- to 10-membered heterocyclyl, wherein the alkyl, alkoxy, cycloalkyl, heterocyclyl, aryl or heteroaryl is optionally further substituted with 0 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl, 5- to 10-membered heteroaryl, 4- to 10-membered heterocyclyl or $R^{b7a}$, and the heteroaryl or heterocyclyl contains 1 to 4 heteroatoms selected from O, S, or N;

$R^{b7a}$ is selected from $C_{1-4}$ alkyl, $-C_{3-6}$ cycloalkyl, 4- to 10-membered heterocyclyl, $-C_{1-4}$ alkylene $-C_{3-6}$ cycloalkyl, $-C_{1-4}$ alkylene 4- to 10-membered heterocyclyl, $-O-C_{3-6}$ cycloalkyl, -O-4- to 10-membered heterocyclyl, $-NH-C_{3-6}$ cycloalkyl, -NH-4- to 10-membered heterocyclyl, $-N(C_{1-4}$ alkyl$)-C_{3-6}$ cycloalkyl or $-N(C_{1-4}$ alkyl)-4- to 10-membered heterocyclyl, wherein the $R^{b7a}$ is optionally substituted with 1 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl or 4- to 10-membered heterocyclyl, and the heterocyclyl contains 1 to 4 heteroatoms selected from O, S, or N;

$R^{b2}$ and $R^{b6}$ are each independently selected from H, F, Cl, Br, I, =O, OH, $-C(=O)N(R^{b21})_2$, $-N(R^{b21})_2$, CN, $CF_3$, C(=O)

OH, $CHF_2$, $C_{1-4}$ alkyl, $C_{1-4}$ alkoxy, $C_{3-6}$ cycloalkyl, $-(CH_2)_n-Rb^{21}$, $-ORb^{21}$, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{1-4}$ alkoxy, $C_{3-6}$ cycloalkyl, 5- to 10-membered heteroaryl or 4- to 10-membered heterocyclyl, and the heteroaryl or heterocyclyl contains 1 to 4 heteroatoms selected from O, S, or N;

each $R^{b21}$ is independently selected from H, $C_{1-6}$ alkyl, $C_{1-4}$ alkoxy, $C_{3-6}$ cycloalkyl, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{3-6}$ cycloalkyl or $C_{1-4}$ alkoxy, and the heteroaryl or heterocyclyl contains 1 to 4 heteroatoms selected from O, S, or N;

n is selected from 0, 1, 2, 3 or 4;

K is selected from

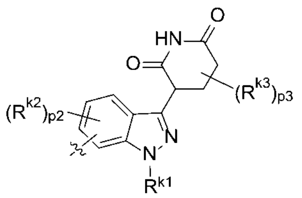,
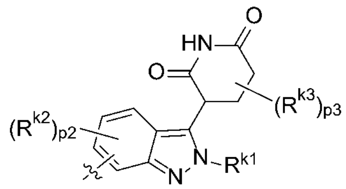,

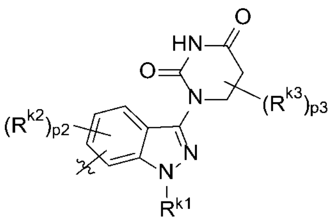 or
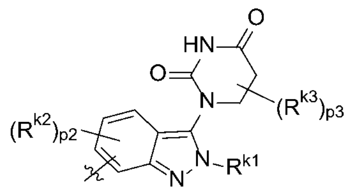;

each $R^{k1}$ is independently selected from H, $C_{1-4}$ alkyl, $C_{2-6}$ alkenyl, $C_{2-6}$ alkynyl, $C_{3-6}$ cycloalkyl or 3- to 6-membered heterocycloalkyl, wherein the alkyl, cycloalkyl or heterocycloalkyl is optionally further substituted with 0 to 4 substituents selected from H, F, Cl, Br, I, OH, $NH_2$, CN, $CF_3$, $C_{1-6}$ alkyl, $C_{1-6}$ alkoxy, $C_{2-6}$ alkenyl, $C_{2-6}$ alkynyl or $C_{3-6}$ cycloalkyl;

$R^{k2}$ and $R^{k3}$ are each independently selected from H, F, Cl, Br, I, OH, =O, $NH_2$, $CF_3$, CN, C(=O)OH, C(=O)$NH_2$, $C_{1-4}$ alkyl or $C_{1-4}$ alkoxy, wherein the alkyl or alkoxy is optionally further substituted with 0 to 4 substituents selected from H, F, Cl, Br, I, OH, or $NH_2$;

or two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, C(=O)OH, C(=O)$NH_2$, $C_{1-4}$ alkyl or $C_{1-4}$ alkoxy, and the heterocycle contains 1 to 4 heteroatoms selected from O, S or N;

q is selected from 0, 1, 2, 3 or 4;

n1, n2 and n6 are each independently selected from 0, 1, 2 or 3;

p2 and p3 are each independently selected from 0, 1, 2, 3 or 4;

optionally, 0 to 50 H of the compound as represented by general formula (I) are replaced by 0 to 50 D;

in certain embodiments, B is selected from

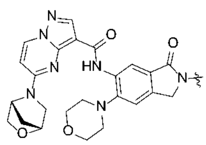,
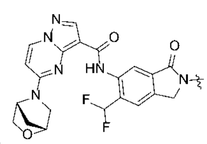,

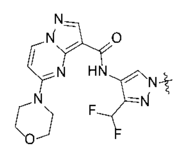,
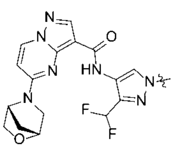,
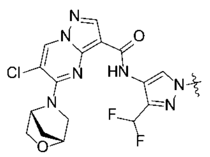,
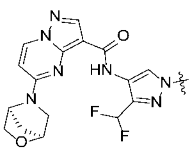,
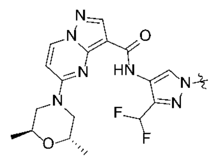,

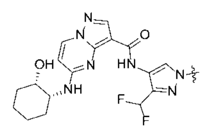, 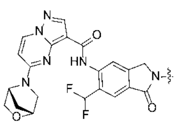, 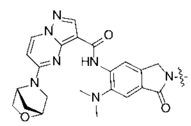, 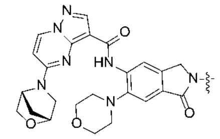, 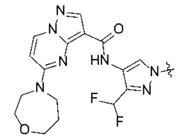,

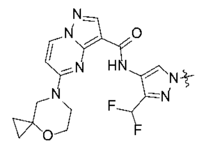, 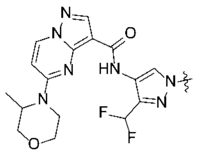, 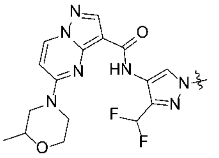, 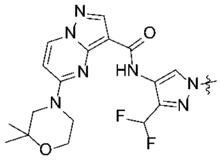,

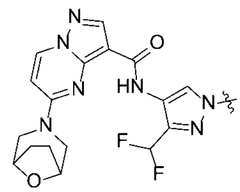, 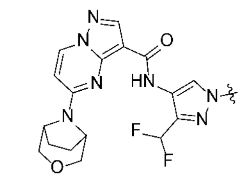, 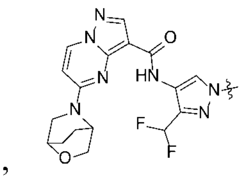, 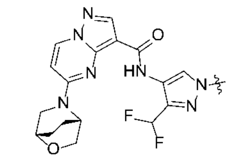,

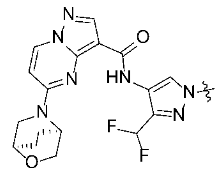, 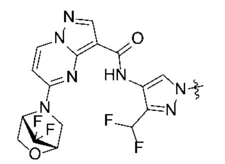, 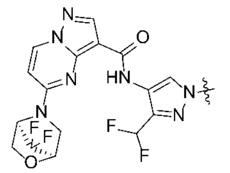, 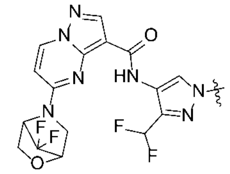,

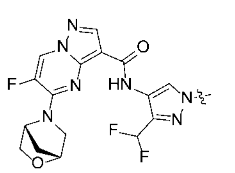, 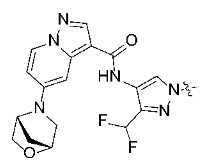, 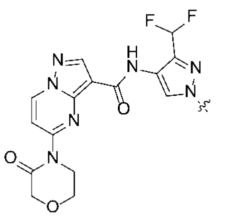, 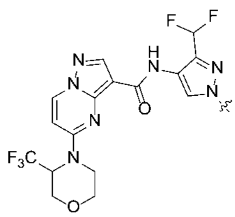,

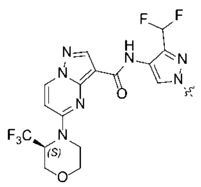, 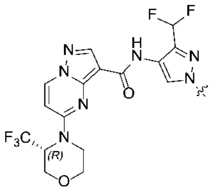, 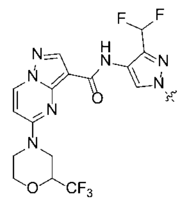, 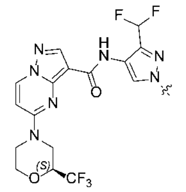, 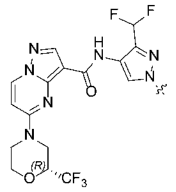,

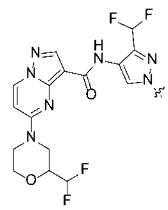, 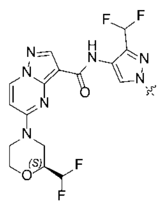, 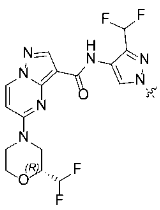, 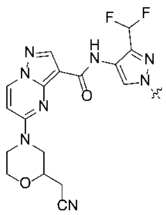, 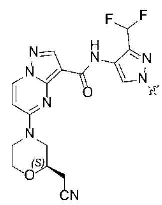, 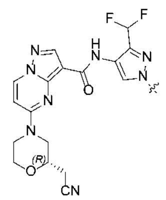,

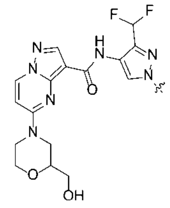, 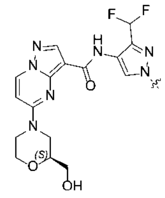, 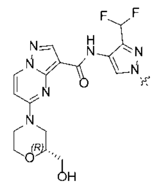, 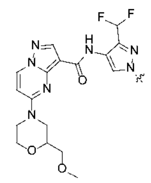, 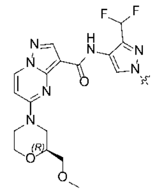, 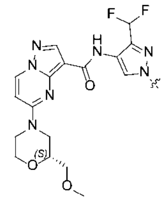,

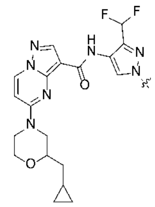, 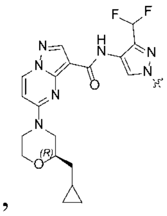, or 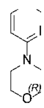,

L is

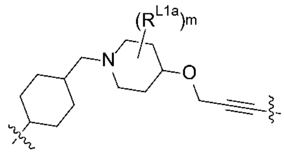,

each $R^{L1a}$ is independently selected from halogen, CN, OH, $C_{1-4}$ alkyl, or $C_{1-4}$ alkoxy, preferably F, Cl, Br, CN, OH, methyl, ethyl, hydroxymethyl, methoxy or ethoxy, m is selected from 0, 1, 2, 3 or 4, preferably, L is selected from

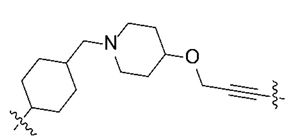, 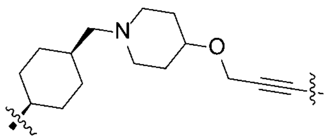, 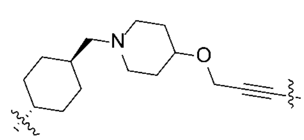,

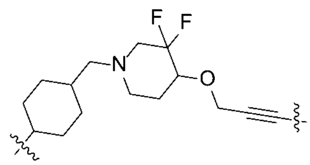, 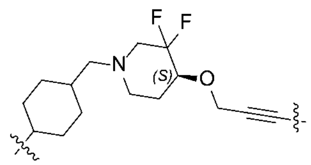, 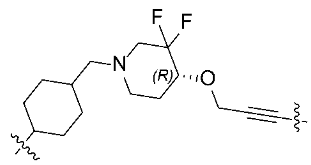,

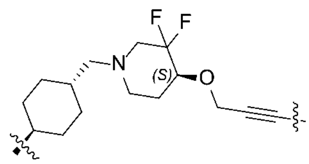, 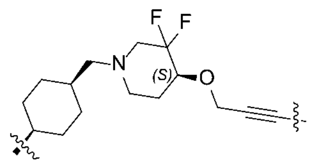, 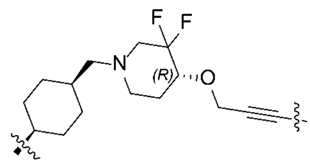,

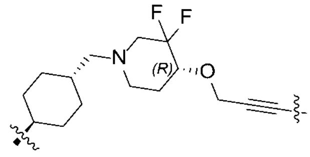, 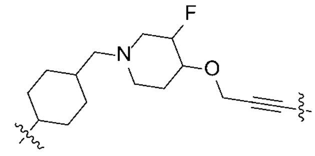, 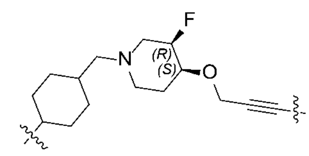,

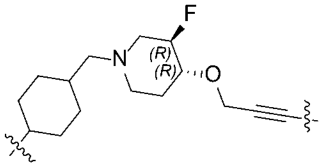, 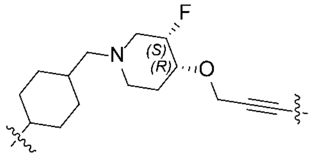, 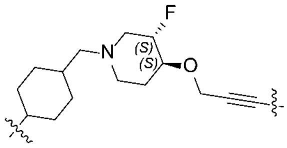,

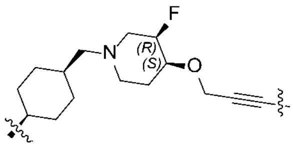, 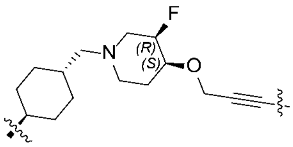, 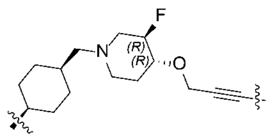,

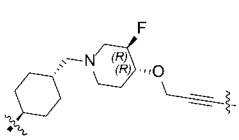, 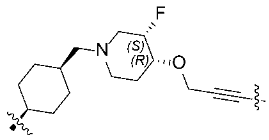, 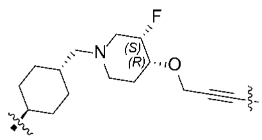, 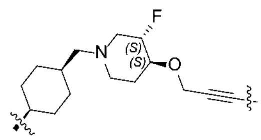

, or 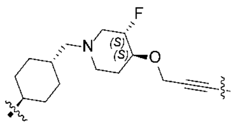,

and K is selected from

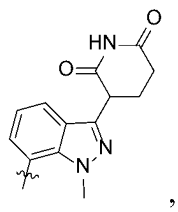, 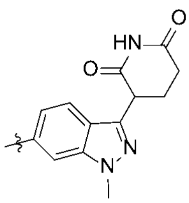, or

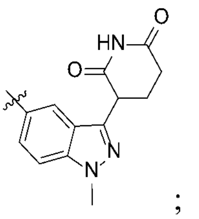;

in certain embodiments, the active ingredient A is selected from a compound as represented by general formula (II) or a stereoisomer, a tautomer, a deuterated substance, a solvate, a prodrug, a metabolite, a pharmaceutically acceptable salt or an eutectic crystal thereof,

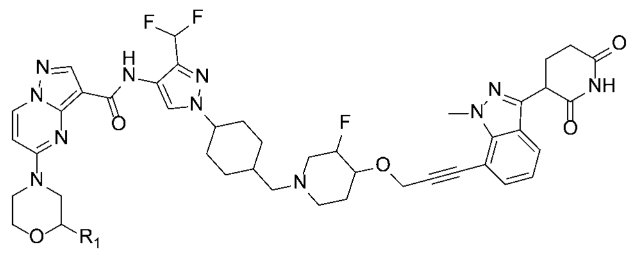

(II);

$R_1$ is selected from $CF_3$, $CHF_2$, $CH_2CF_3$, $CH_2OH$, $CH_2OMe$, $CH_2CN$, or $CH_2$-cyclopropyl;

in certain embodiments, the structure of the compound as represented by general formula (I) is selected from one of

the structures shown in Table S-1;

Table S-1 Compound structures

| | |
|---|---|
| 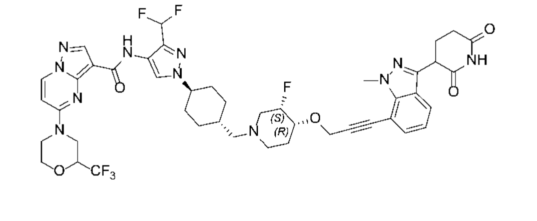 | 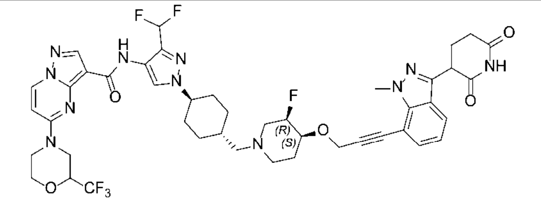 |

| | |
|---|---|
| 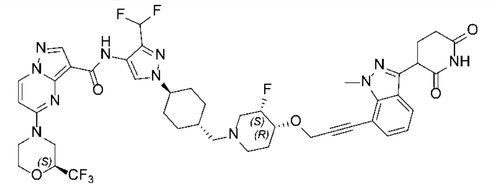 | 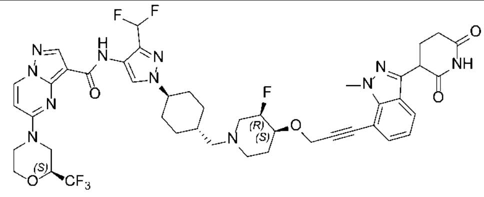 |
| 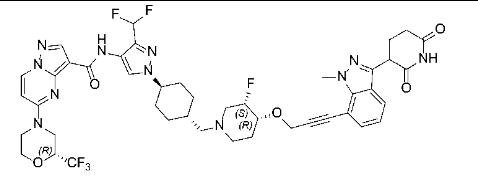 | 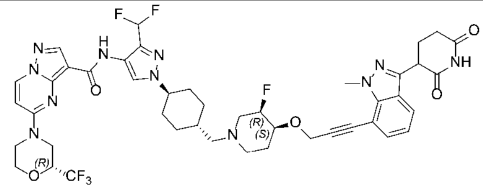 |
| 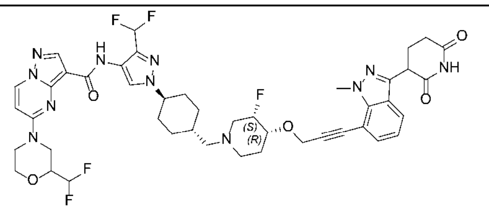 | 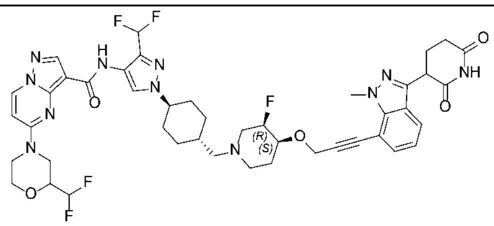 |
| 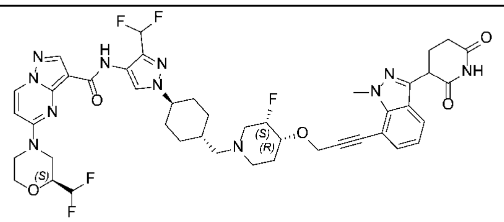 | 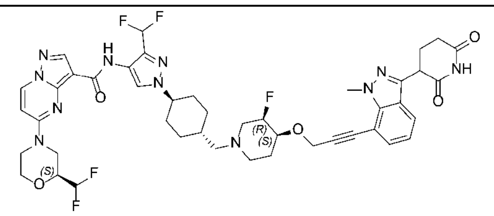 |
| 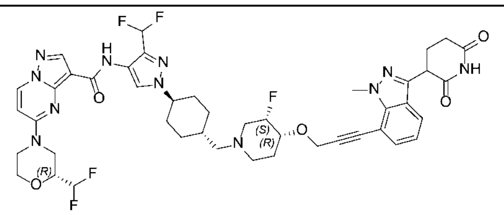 | 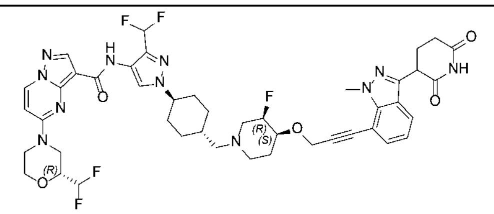 |
| 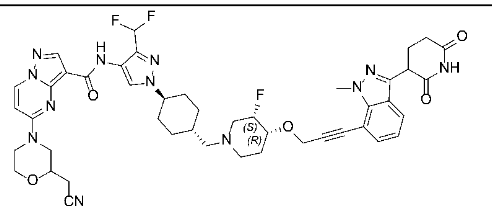 | 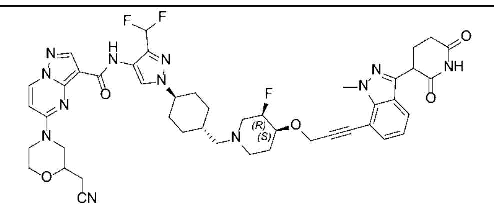 |
| 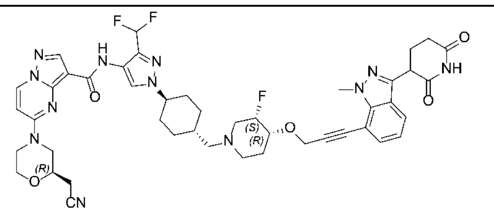 | 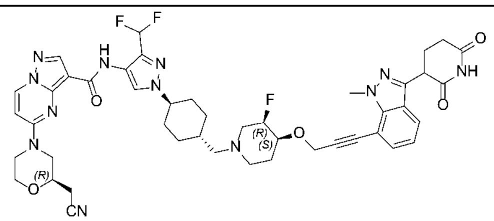 |
| 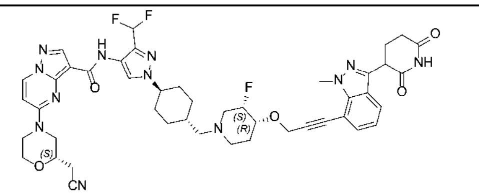 | 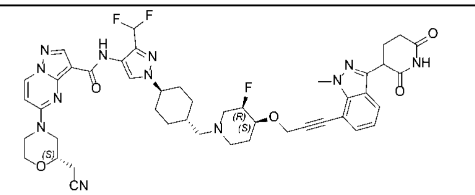 |

| | |
|---|---|
| 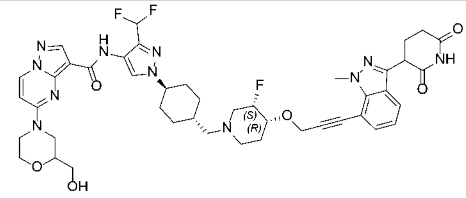 | 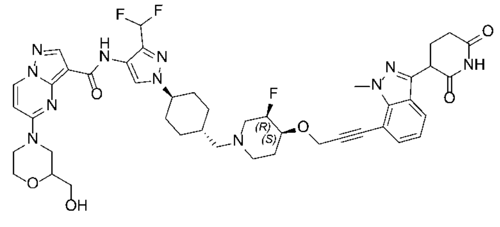 |
| 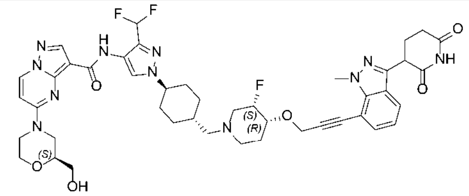 | 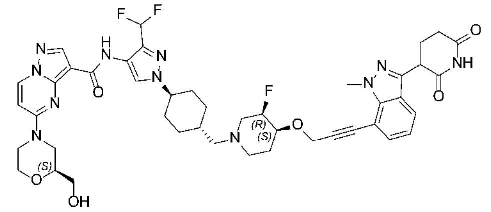 |
| 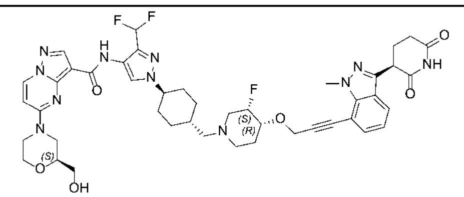 | 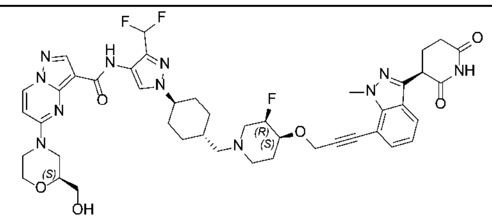 |
| 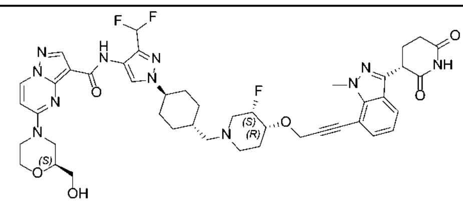 | 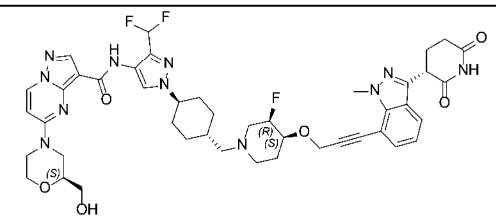 |
| 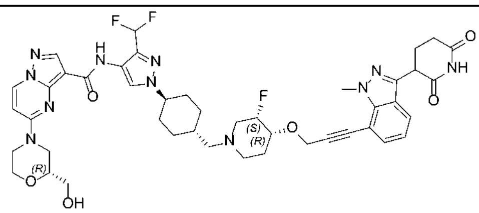 | 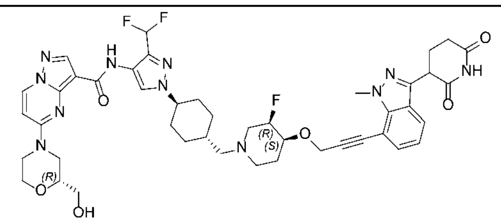 |
| 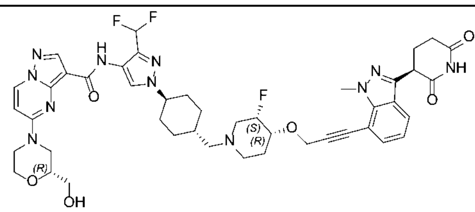 | 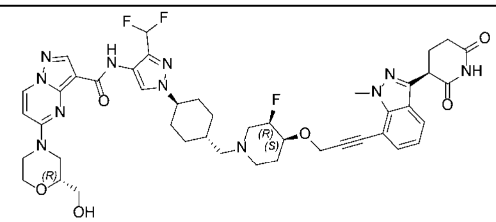 |
| 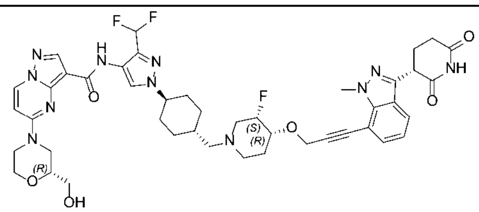 | 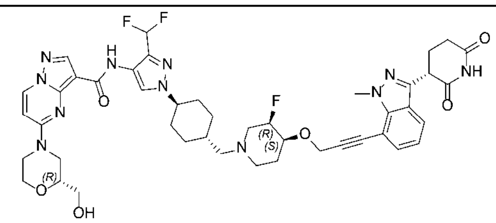 |

| | |
|---|---|
| 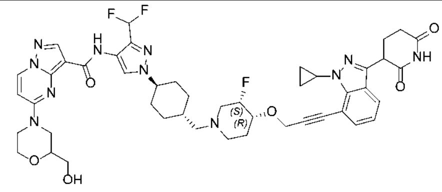 | 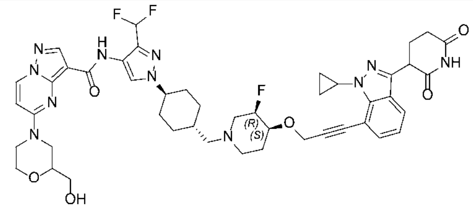 |
| 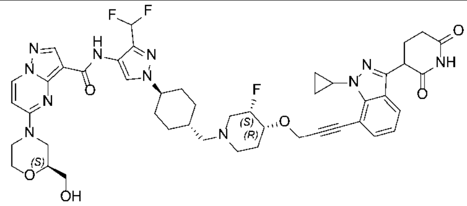 | 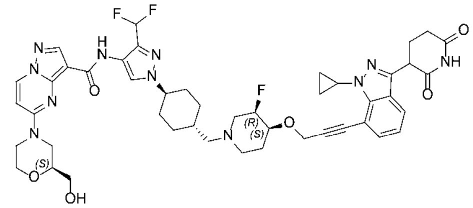 |
| 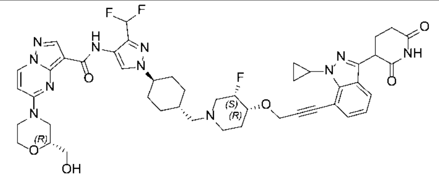 | 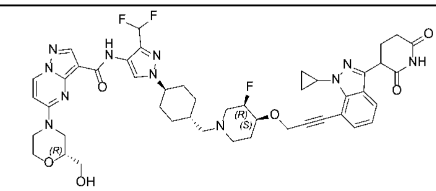 |
| 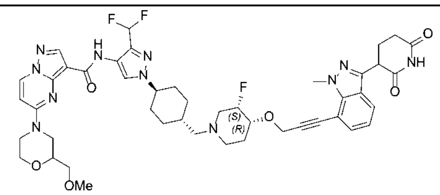 | 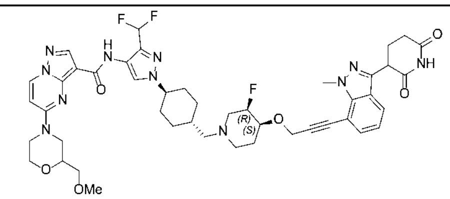 |
| 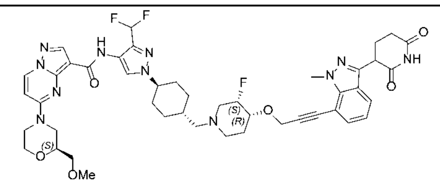 | 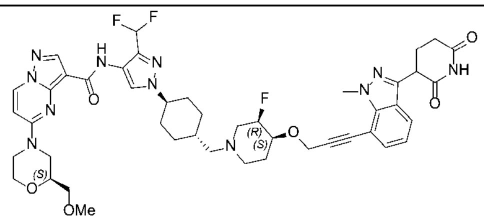 |
| 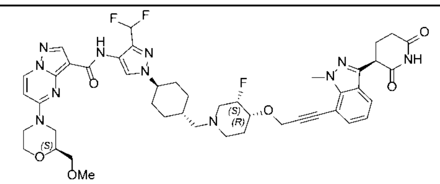 | 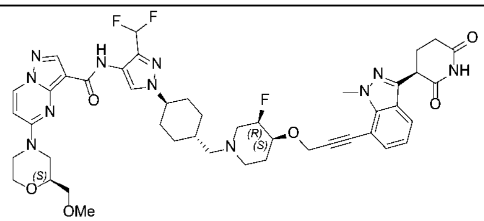 |
| 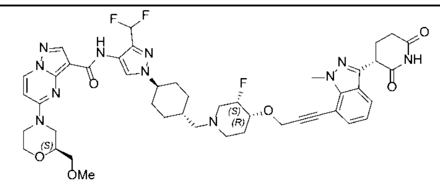 | 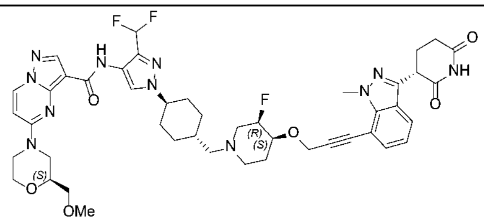 |
| 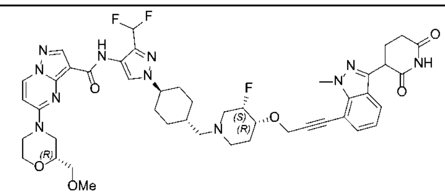 | 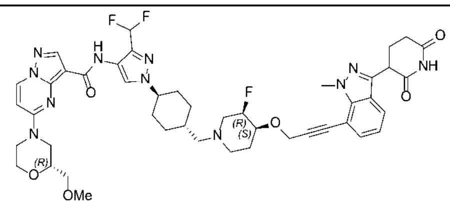 |

| | |
|---|---|
| 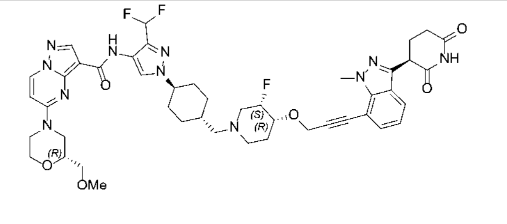 | 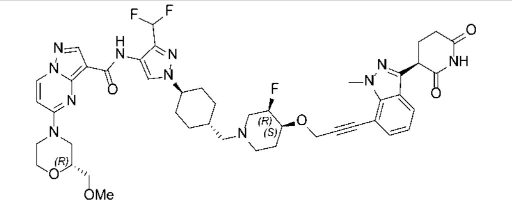 |
| 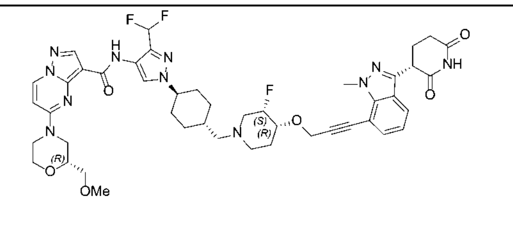 | 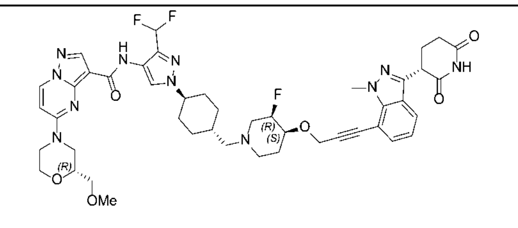 |
| 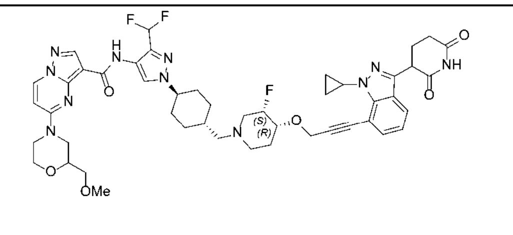 | 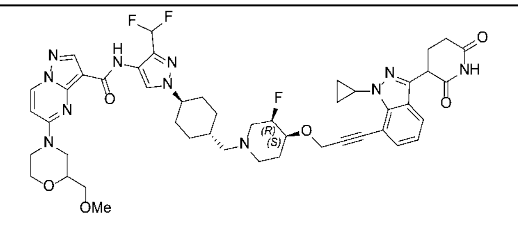 |
| 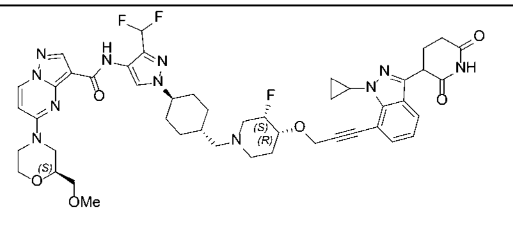 | 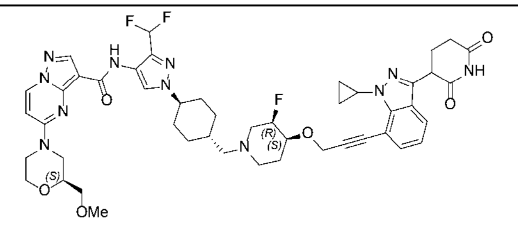 |
| 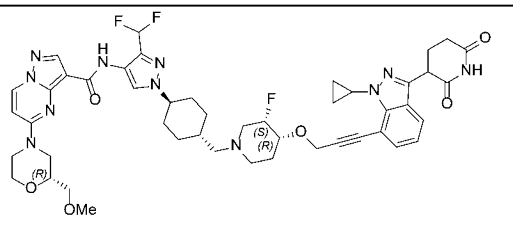 | 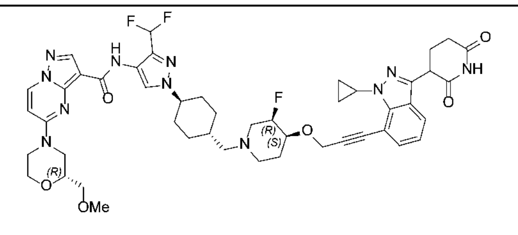 |
| 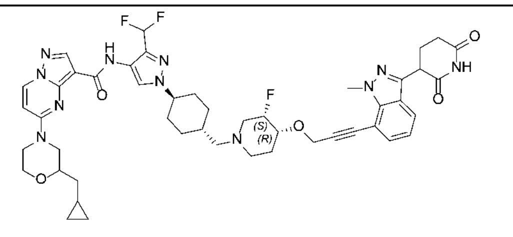 | 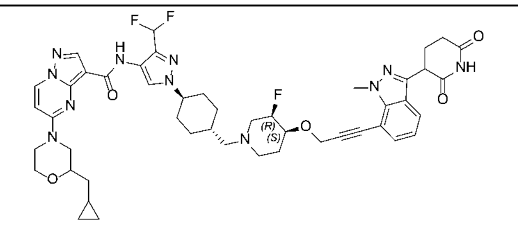 |
| 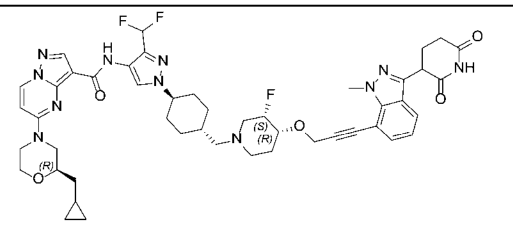 | 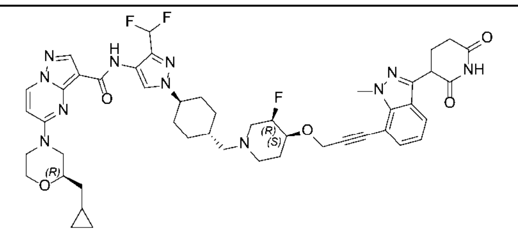 |

| | |
|---|---|
| 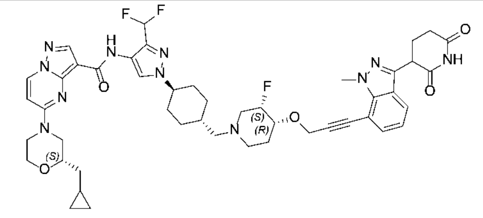 | 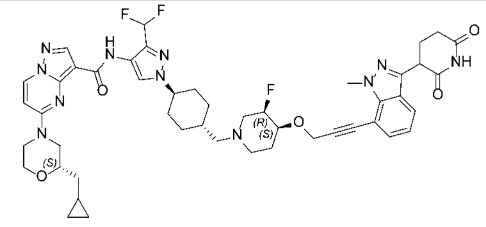 |
| 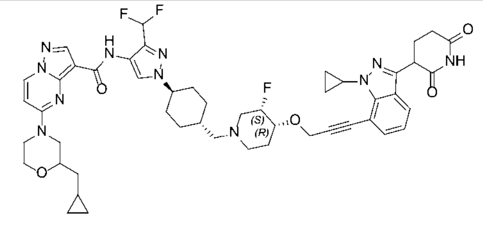 | 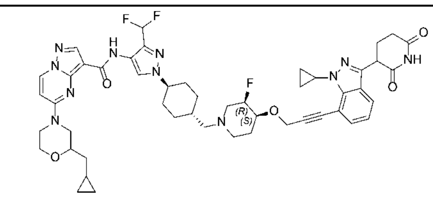 |
| 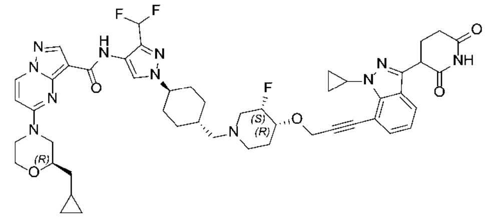 | 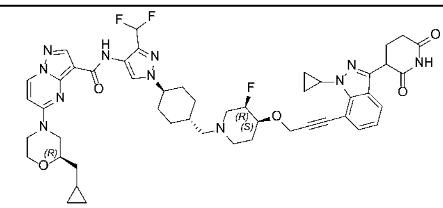 |
| 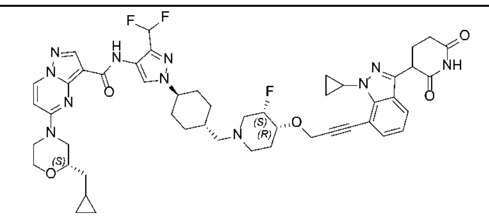 | 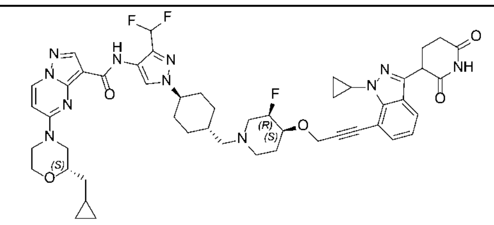 |
| 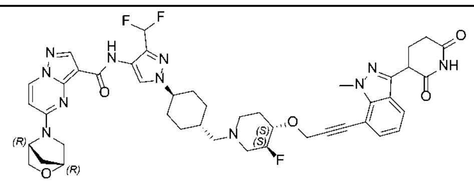 | |

;

in some embodiments, the compound as represented by general formula (I) is selected from the following compounds:

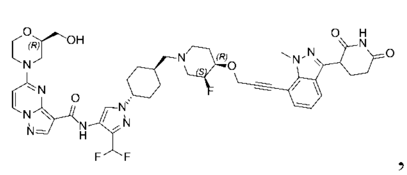 , 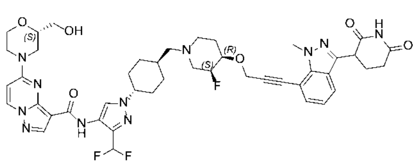 ,

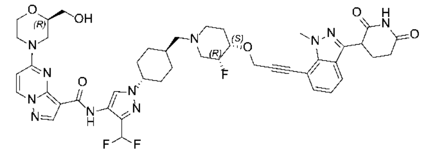 , 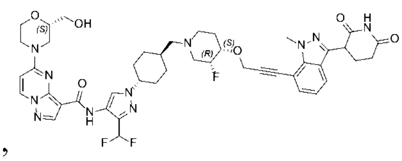 .

In one aspect, the pharmaceutical composition comprises 0.5-800 mg of the active ingredient A;

in some embodiments, the pharmaceutical composition comprises 1-600 mg of the active ingredient A;
in some embodiments, the pharmaceutical composition comprises 1-400 mg of the active ingredient A;

in some embodiments, the pharmaceutical composition comprises 5-600 mg of the active ingredient A;
in some embodiments, the pharmaceutical composition comprises 5-350 mg of the active ingredient A;
in some embodiments, the pharmaceutical composition comprises 10-600 mg of the active ingredient A;
in some embodiments, the pharmaceutical composition comprises 10-400 mg of the active ingredient A;
in some embodiments, the pharmaceutical composition comprises 20-400 mg of the active ingredient A;
in some embodiments, the pharmaceutical composition comprises 30-300 mg of the active ingredient A;
in some embodiments, the pharmaceutical composition comprises 25-200 mg of the active ingredient A;
in some embodiments, the pharmaceutical composition comprises 20-200 mg of the active ingredient A;
in some embodiments, the pharmaceutical composition comprises 10-200 mg of the active ingredient A;
in some embodiments, the pharmaceutical composition comprises 30-100 mg of the active ingredient A;
in some embodiments, the pharmaceutical composition comprises 50-100 mg of the active ingredient A;
in some embodiments, the pharmaceutical composition comprises 5-100 mg of the active ingredient A;
in some embodiments, the pharmaceutical composition comprises 5 mg of the active ingredient A;
in some embodiments, the pharmaceutical composition comprises 10 mg of the active ingredient A;
in some embodiments, the pharmaceutical composition comprises 20 mg of the active ingredient A;
in some embodiments, the pharmaceutical composition comprises 25 mg of the active ingredient A;
in some embodiments, the pharmaceutical composition comprises 30 mg of the active ingredient A;
in some embodiments, the pharmaceutical composition comprises 40 mg of the active ingredient A;
in some embodiments, the pharmaceutical composition comprises 50 mg of the active ingredient A;
in some embodiments, the pharmaceutical composition comprises 75 mg of the active ingredient A;
in some embodiments, the pharmaceutical composition comprises 100 mg of the active ingredient A;
in some embodiments, the pharmaceutical composition comprises 125 mg of the active ingredient A;
in some embodiments, the pharmaceutical composition comprises 150 mg of the active ingredient A;
in some embodiments, the pharmaceutical composition comprises 200 mg of the active ingredient A;
in some embodiments, the pharmaceutical composition comprises 300 mg of the active ingredient A;
in some embodiments, the pharmaceutical composition comprises 400 mg of the active ingredient A;
in some embodiments, the pharmaceutical composition comprises 500 mg of the active ingredient A;
in some embodiments, the pharmaceutical composition comprises 600 mg of the active ingredient A;
in some embodiments, the pharmaceutical composition comprises 700 mg of the active ingredient A;
in some embodiments, the pharmaceutical composition comprises 800 mg of the active ingredient A.

In another aspect, the content of the active ingredient A in the aforementioned pharmaceutical composition is selected from 0.5%-99.0%;

in some embodiments, the content of the active ingredient A is 0.5%-99.0%; in some embodiments, the content of the active ingredient A is 1.0%-80.0%; in some embodiments, the content of the active ingredient A is 1.0%-70.0%; in some embodiments, the content of the active ingredient A is 1.0%-60.0%; in some embodiments, the content of the active ingredient A is 1.0%-50.0%; in some embodiments, the content of the active ingredient A is 1.0%-40.0%; in some embodiments, the content of the active ingredient A is 1.0%-30.0%; in some embodiments, the content thereof is 1.0%-20.0%; in some embodiments, the content of the active ingredient A is 1.0%-10.0%; in some embodiments, the content of the active ingredient A is 2.0%-90.0%; in some embodiments, the content of the active ingredient A is 2.0%-85.0%; in some embodiments, the content of the active ingredient A is 2.0%-70.0%; in some embodiments, the content of the active ingredient A is 2.0%-60.0%; in some embodiments, the content of the active ingredient A is 2.0%-50.0%; in some embodiments, the content of the active ingredient A is 2.0%-40.0%; in some embodiments, the content of the active ingredient A is 2.0%-30.0%; in some embodiments, the content of the active ingredient A is 2.0%-20.0%; in some embodiments, the content of the active ingredient A is 2.0%-10.0%; in some embodiments, the content of the active ingredient A in the pharmaceutical composition is 1%-50%, 2%-30%, 2%-20%, or 2%-18%; in some embodiments, the content of the active ingredient A is 2.7%; in some embodiments, the content of the active ingredient A is 9.5%; in some embodiments, the content of the active ingredient A is 9.8%; in some embodiments, the content of the active ingredient A is 14.5%; and in some embodiments, the content of the active ingredient A is 16.7%.

In another aspect, the pharmaceutically acceptable excipient in the aforementioned pharmaceutical composition contains one or both of a solubilizer or a solid dispersion carrier;

in some embodiments, an amorphous solid dispersion is prepared with the active ingredient A and the solid dispersion carrier;
in some embodiments, an amorphous solid dispersion is prepared with the active ingredient A, the solid dispersion carrier and the solubilizer.

In some embodiments, the solubilizer is selected from one or more of anionic surfactant, cationic surfactant,

zwitterionic surfactant, nonionic surfactant, polymeric surfactant or cyclodextrin;
in some embodiments, the solubilizer is selected from sodium lauryl sulphate (SLS), vitamin E polyethylene glycol succinate (TPGS), polyethylene glycol and a derivative thereof, cyclodextrin or polyoxyethylene hydrogenated castor oil. In some embodiments, the weight ratio of the active ingredient A to the solubilizer is 1 : 0.01-1 : 100, preferably 1 : 0.05-1 : 30, and further preferably 1 : 0.1-1 : 20.

In some embodiments, the solid dispersion carrier is selected from polymer carriers, preferably one or more of hydroxypropyl methylcellulose derivatives, ethyl celluloses, methyl celluloses, hydroxypropyl methylcellulose acetate succinate, povidones, copovidone, polyethylene glycols with an average molecular weight of not less than 1000, hydroxypropyl cellulose, polyvinyl caprolactam-polyvinyl acetate-polyethylene glycol graft polymers, polyacrylic resin, hydroxypropyl methylcellulose phthalate, or polyvinyl alcohol, and more preferably one or more of copovidone, hydroxypropyl cellulose, hydroxypropyl methylcellulose, Eudragit, hydroxypropyl methylcellulose acetate succinate (HPMCAS), or hydroxypropyl methylcellulose phthalate (HPMCP); and the weight ratio of the active ingredient A to the solid dispersion carrier is 1 : 0.01-1 : 100, preferably 1 : 0.5-1 : 10, and further preferably 1 : 1-1 : 6.

In some embodiments, the solid dispersion carrier is selected from Eudragit, hydroxypropyl methylcellulose phthalate (HPMCP) or hydroxypropyl methylcellulose acetate succinate (HPMCAS), and the weight ratio of the active ingredient A to the solid dispersion carrier is 1 : 1-1 : 6, preferably 1 : 1-1 : 4, more preferably 1 : 2 or 1 : 3, and even more preferably 1 : 3 or 1 : 4;

in some embodiments, the solid dispersion carrier is Eudragit L100;
in some embodiments, the solid dispersion carrier is selected from Eudragit or hydroxypropyl methylcellulose phthalate (HPMCP), and the weight ratio of the active ingredient A to the solid dispersion carrier is 1 : 1-1 : 6, preferably 1 : 1-1 : 4, and more preferably 1 : 3 or 1 : 4;
in some embodiments, the solid dispersion carrier is hydroxypropyl methylcellulose acetate succinate (HPMCAS), the weight ratio of the active ingredient A to the solid dispersion carrier is 1 : 1-1 : 6, preferably 1 : 1-1 : 4, more preferably 1 : 2, and even more preferably 1 : 3 or 1 : 4, the solubilizer is vitamin E polyethylene glycol succinate (TPGS), and the weight ratio of the active ingredient A to the solubilizer is 1 : 0.05-1 : 20, further preferably 1 : 0.1-1 : 10, and more preferably 1 : 0.3 or 1 : 0.33; and in some embodiments, a solid dispersion, as an intermediate of a pharmaceutical formulation, is combined with other pharmaceutically acceptable excipient to prepare a finished formulation.

In some embodiments, the solid dispersion is further combined with other pharmaceutically acceptable excipients;

in some embodiments, the content of the solid dispersion in the pharmaceutical composition is 10%-100%, preferably 15%-65%, further preferably 25-55%, and even further preferably 20%-60%;
in some embodiments, the content of the solid dispersion in the pharmaceutical composition is 10%-100%, preferably 20%-70%, and further preferably 40%-60%.

In some embodiments, the pharmaceutically acceptable excipient further contains one or more of a filler, a disintegrant, a binder, a suspending agent, an antioxidant, a colorant, a glidant, and a lubricant;

in some embodiments, the pharmaceutically acceptable excipient contains a filler or a lubricant;
in some embodiments, the pharmaceutically acceptable excipient contains a filler, a binder, or a lubricant;
in some embodiments, the pharmaceutically acceptable excipient contains a filler, a binder, a disintegrant, a lubricant or a glidant;
in some embodiments, the pharmaceutically acceptable excipient contains a filler, a disintegrant, a lubricant or a glidant;
in some embodiments, the filler in any aforementioned solution is selected from one or more of lactose, microcrystalline cellulose, sucrose, glucose, powdered cellulose, calcium phosphate, dicalcium phosphate, calcium carbonate, aluminium silicate, dextrin, starch (including corn starch, potato starch or amylopectin), pregelatinized starch, sodium chloride, potassium chloride, mannitol or sorbitol, preferably one or more of lactose, microcrystalline cellulose, dicalcium phosphate, pregelatinized starch, or mannitol, and more preferably one or more of lactose, microcrystalline cellulose, dicalcium phosphate, or mannitol; optionally, the filler is lactose; optionally, the filler is dicalcium phosphate; optionally, the filler is selected from lactose or microcrystalline cellulose; optionally, the filler is microcrystalline cellulose; optionally, the filler is mannitol; optionally, the filler is selected from mannitol or microcrystalline cellulose, wherein the content of lactose in the pharmaceutical composition is selected from 20%-60%, preferably 30%-55%, 34.5%, 35.6%, or 53.7%; in some embodiments, the content of dicalcium phosphate in the pharmaceutical composition is selected from 10%-20%, preferably 13.3%; in some embodiments, the content of microcrystalline cellulose in the pharmaceutical composition is selected from 5%-40%, preferably 10%-40%, and more preferably

17.2%, 17.7%, or 38.8%; in some embodiments, the content of mannitol in the pharmaceutical composition is selected from 5%-40%, preferably 8.1%;

in some embodiments, the disintegrant in any aforementioned solution is selected from one or more of starch, pregelatinized starch, carboxymethyl starch sodium, crospovidone, or croscarmellose sodium, preferably one or more of carboxymethyl starch sodium, crospovidone, or croscarmellose sodium, and more preferably crospovidone and croscarmellose sodium; in some embodiments, the disintegrant is selected from crospovidone and croscarmellose sodium, wherein the content of crospovidone in the pharmaceutical composition is selected from 1%-10%, preferably 3.1%, 3.2%, or 5.8%; and the content of croscarmellose sodium in the pharmaceutical composition is selected from 1%-10%, preferably 3.1%, 3.2%, or 5.8%;

in some embodiments, the binder in any aforementioned solution is selected from one or more of starch slurry, povidone, hydroxypropyl cellulose, hydroxypropyl methylcellulose, ethyl cellulose, sodium carboxymethyl cellulose, xanthan gum, arabic gum, gelatine, guar gum or carbomer, preferably one or more of povidone, hydroxypropyl cellulose, or hydroxypropyl methylcellulose, and more preferably one or more of povidone, hydroxypropyl methylcellulose E3, or hydroxypropyl methylcellulose E5; optionally, the binder is hydroxypropyl methylcellulose E5; optionally, the binder is povidone, wherein the content of hydroxypropyl methylcellulose E5 in the pharmaceutical composition is selected from 1%-5%, preferably 1.3%; and the content of povidone in the pharmaceutical composition is selected from 1%-5%, preferably 3.1%;

in some embodiments, the suspending agent in any aforementioned solution is selected from one or more of arabic gum, tragacanth gum, xanthan gum, peach gum, sodium alginate, agar, starch slurry, methyl cellulose, sodium carboxymethyl cellulose, hydroxypropyl cellulose, carbomer, povidone, dextran, aluminium monostearate, or aluminium silicate, preferably one or more of xanthan gum, sodium alginate, methyl cellulose or hydroxypropyl cellulose, more preferably xanthan gum or hydroxypropyl cellulose; optionally, the suspending agent is xanthan gum, wherein the content of xanthan gum in the pharmaceutical composition is selected from 1%-5%, preferably 3%;

in some embodiments, the glidant in any aforementioned solution is selected from one or more of talc and colloidal silicon dioxide, preferably colloidal silicon dioxide; and in some embodiments, the content of colloidal silicon dioxide in the pharmaceutical composition is selected from 0.1%-2%, preferably 1%.

In some embodiments, the lubricant in any aforementioned solution is selected from one or more of stearic acid, magnesium stearate, or sodium stearyl fumarate, preferably magnesium stearate or sodium stearyl fumarate; in some embodiments, the content of magnesium stearate in the pharmaceutical composition is selected from 0.1%-3%, preferably 1% or 1.2%; and in some embodiments, the content of sodium stearyl fumarate in the pharmaceutical composition is selected from 0.1%-3%, preferably 1.5%.

Optionally, the pharmaceutical composition further comprises one or more of a flavouring agent, an antioxidant, a preservative, an opacifier, and a film coating premixer.

in some embodiments, the binder in the pharmaceutical composition as described above can be added in a solution or powder form; and the disintegrant can be added internally, externally, or both.

The active ingredient A in any one of the above-mentioned pharmaceutical compositions is selected from a compound as represented by formula (I) or a stereoisomer, a tautomer, a deuterated substance, a solvate, a prodrug, a metabolite, a pharmaceutically acceptable salt or an eutectic crystal thereof.

In one aspect, the present invention provides a pharmaceutical formulation, comprising any one of the above-mentioned pharmaceutical compositions.

In some embodiments, the formulation form of the pharmaceutical formulation is selected from a tablet, a granule, a capsule, dry suspension, a powder, and an oral solution.

The preparation process of the pharmaceutical formulation of the present invention is one or more of direct mixing, wet granulation, dry granulation, fluidized bed granulation, spray drying, freeze drying, and hot melt extrusion, preferably direct mixing, wet granulation, fluidized bed granulation, dry granulation and spray drying, and more preferably wet granulation, dry granulation, and spray drying.

In some embodiments, the preparation process is one or more of direct mixing, wet granulation, dry granulation, and spray drying, preferably spray drying;

in some embodiments, the preparation process is wet granulation;

in some embodiments, the preparation process is dry granulation.

The present invention also provides the use of the pharmaceutical composition in the preparation of a relevant drug for treating tumours or autoimmune system diseases.

Unless otherwise stated, the terms used in the description and claims of the present application have the following meanings.

The term "formulation strength" refers to the weight of the active drug (active ingredient A) contained in each vial,

tablet, or other unit formulation.

The carbon, hydrogen, oxygen, sulphur, nitrogen or F, Cl, Br, I involved in the groups and compounds of the present invention all comprise their isotopes, and the carbon, hydrogen, oxygen, sulphur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulphur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"CN" refers to cyano.

"Halogen" refers to F, Cl, Br or I.

"Halogen-substituted" refers to substitution with F, Cl, Br, or I, including but not limited to substitution with 1 to 10 substituents selected from F, Cl, Br, or I, or substitution with 1 to 6 substituents selected from F, Cl, Br, or I, or substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbyl group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. Alkyl can be monovalent, divalent, trivalent or tetravalent.

"Heteroalkyl" refers to a substituted or unsubstituted alkyl group in which one or more (including but not limited to 2, 3, 4, 5 or 6) carbon atoms are replaced by heteroatoms (including but not limited to N, O or S). Non-limiting examples include $-X-(CH_2)v-X-(CH_2)v-X-(CH_2)v-H$ (v is an integer from 1 to 5; each X is independently selected from a bond or a heteroatom, which includes but is not limited to N, O or S; at least one X is selected from a heteroatom; and N or S in the heteroatom can be oxidized to various oxidation states). Heteroalkyl can be monovalent, divalent, trivalent or tetravalent.

"Alkylene" refers to a substituted or unsubstituted linear or branched divalent saturated hydrocarbyl group, including $-(CH_2)_v-$ (v is an integer from 1 to 10), and examples of alkylene include, but are not limited to, methylene, ethylene, propylene, butylene, *etc.*

"Heteroalkylene" refers to a substituted or unsubstituted alkylene group in which one or more (including but not limited to 2, 3, 4, 5 or 6) carbon atoms are replaced by heteroatoms (including but not limited to N, O or S). Non-limiting examples include $-X-(CH_2)v-X-(CH_2)v-X-(CH_2)v-$, wherein v is an integer from 1 to 5, each X is independently selected from a bond, N, O or S, and at least one X is selected from N, O or S.

"Cycloalkyl" refers to a substituted or unsubstituted saturated carbocyclic hydrocarbon group, typically having 3 to 12 carbon atoms, and the cycloalkyl can be monocyclic, fused, bridged and spirocyclic. Non-limiting examples include cyclopropyl, cyclobutyl, cyclopentyl, cyclohexyl, cycloheptyl, cyclobutyl fused cyclobutyl, cyclobutyl spiro cyclobutyl, adamantane, etc. Cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 12 atoms, or 3 to 8 atoms, wherein 1 to 3 heteroatoms are included and selected from N, O, S or Se. The C, N and S in the ring of the heterocycloalkyl can be oxidized to various oxidation states. The heterocycloalkyl can be monocyclic, fused, bridged and spirocyclic. The heterocycloalkyl can be connected to a heteroatom or a carbon atom, and non-limiting examples include oxiranyl, aziridinyl, oxetanyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl, piperazinyl,

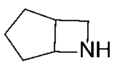, 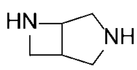, 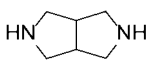,

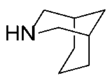, 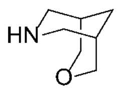, 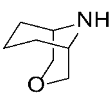, 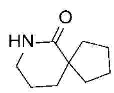, and 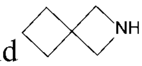.

The heterocycloalkyl can be monovalent, divalent, trivalent or tetravalent.

"Alkenyl" refers to a substituted or unsubstituted linear or branched unsaturated hydrocarbyl group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. Alkenyl can be monovalent, divalent, trivalent or tetravalent.

"Alkynyl" refers to a substituted or unsubstituted linear or branched unsaturated hydrocarbyl group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, *etc.* Alkynyl can be monovalent, divalent, trivalent or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system. The carbocyclyl can be connected to an aromatic ring or non-aromatic ring, and the ring is optionally monocyclic, fused, bridged or spirocyclic. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

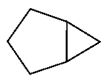, 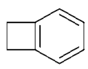, 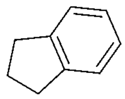

or

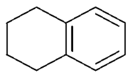.

"Carbocyclyl" or "carbocycle" can be monovalent, divalent, trivalent or tetravalent.

"Heterocyclyl" or "heterocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system, and contains 1 or more (including but not limited to 2, 3, 4 or 5) heteroatoms selected from N, O, S or Se, and the selectively substituted C, N, S or Se in the ring of the heterocyclyl can be oxidized to various oxidation states. The heterocyclyl can be connected to a heteroatom or a carbon atom, can be connected to an aromatic ring or a non-aromatic ring, and is optionally monocyclic, bridged, fused or spirocyclic. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

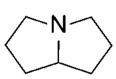,

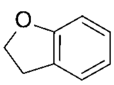, 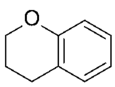, 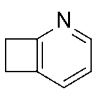, 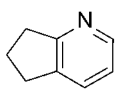, 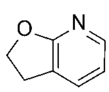, 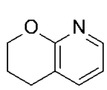, 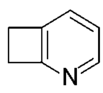, 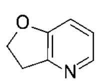,

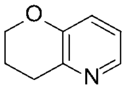, 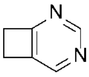, 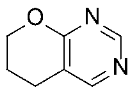, 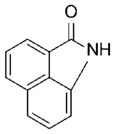, 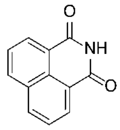, 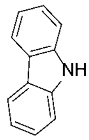, 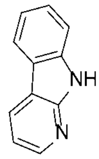, 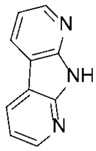,

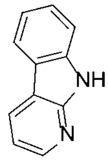, 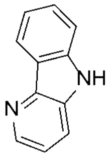, 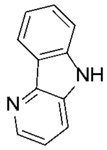, 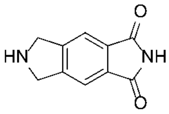, and 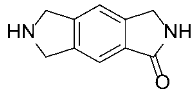.

"Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and may optionally contain 0 to 5 heteroatoms selected from N, O or $S(=O)_n$ (n is 0, 1, or 2).

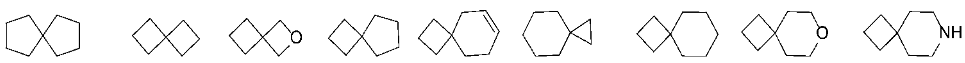

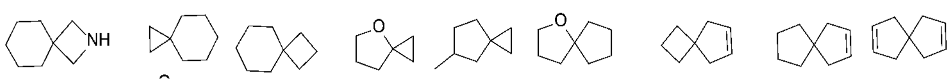

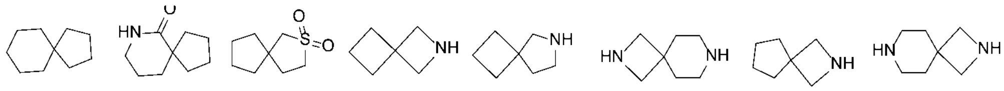

. "Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes but is not limited to 5 to 20, 5 to 14, 5 to 12, or 5 to 10. Non-limiting examples include:

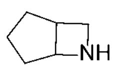,

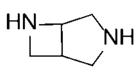, 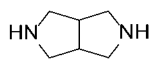,

or

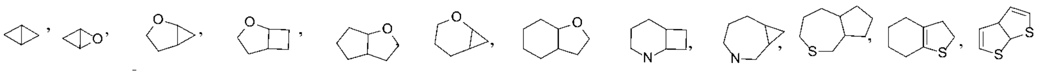

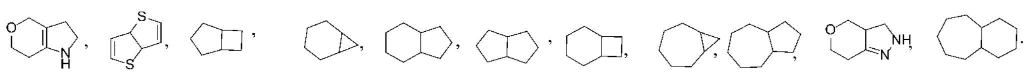

.

"Fused ring" or "fused ring group" can be monovalent, divalent, trivalent or tetravalent.

"Bridged ring" or "bridged ring group" means a substituted or unsubstituted polycyclic group containing any two atoms that are not directly connected, and may contain 0 or more double bonds. Any ring in the bridged ring system may contain 0 to 5 groups selected from heteroatoms or groups containing heteroatoms (including but not limited to N, S(=O)n

or O, wherein n is 0, 1 or 2). The number of ring atoms includes but is not limited to 5 to 20, 5 to 14, 5 to 12 or 5 to 10. Non-limiting examples include

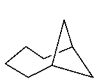, 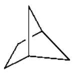, 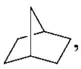, 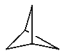, 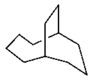, 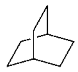, 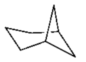, 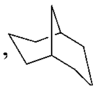, 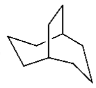, 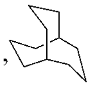,

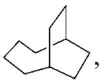, 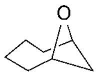, 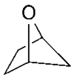, 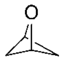, 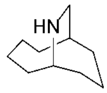, 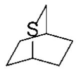, 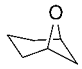, 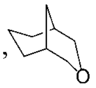, 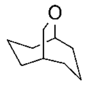, 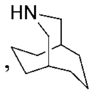,

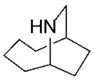, 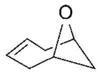, 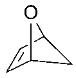, 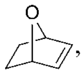, 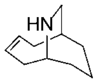, 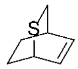, 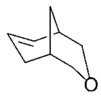, 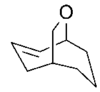, 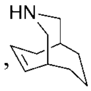,

cubane, and adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused-heterocyclic ring", "fused-heterocyclyl", "fused ring heterocyclyl" or "fused-heterocyclic ring group" refers to a "fused ring" containing a heteroatom.

"Spiro-heterocyclic ring", "spiro-heterocyclyl", "spiro ring heterocyclyl" or "spiro-heterocyclic ring group" refers to a "spiro ring" containing a heteroatom.

"Bridged-heterocyclic ring", "bridged-heterocyclyl", "bridged ring heterocyclyl" or "bridged-heterocyclic ring group" refers to a "bridged ring" containing a heteroatom.

"Aryl" or "aromatic ring" refers to a substituted or unsubstituted aromatic hydrocarbyl group with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocyclic ring, wherein the ring connected to the parent structure is an aryl ring. Non-limiting examples include a benzene ring, a naphthalene ring, and

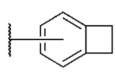, 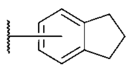.

The "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O, S(=O)n or Se(=O)n, wherein n is 0, 1 or 2), wherein the number of ring atoms in the heteroaromatic ring includes, but is not limited to, 5 to 15, 5 to 10, or 5 to 6. The atoms C, N and S in the ring are optionally oxidized (i.e., C(=O), NO, S(=O)n, and Se(=O)n, wherein n is 1 or 2). Non-limiting examples of heteroaryl include, but are not limited to pyridyl, furyl, thienyl, selenophenyl, pyridyl, pyranyl, N-alkylpyrrolyl, pyrimidyl, pyrazinyl, pyridazinyl, imidazolyl, benzopyrazolyl, benzimidazolyl, benzopyridyl, pyrrolopyridyl, pyridonyl, etc. The heteroaryl ring can be fused to a saturated or unsaturated carbocyclic ring or heterocyclic ring, wherein the ring connected to the parent structure is an aryl ring. Non-limiting examples include

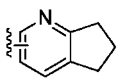, 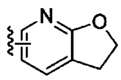, 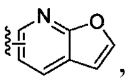, 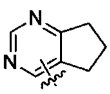, 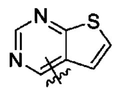, 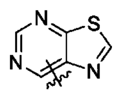, 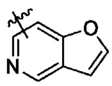,

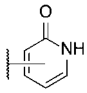, and 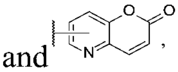, 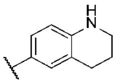, 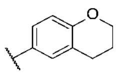.

The definition of the "heteroaryl" herein is consistent with this definition. Heteroaryl can be monovalent, divalent, trivalent or tetravalent. When divalent, trivalent or tetravalent, the points of connection are on the aromatic ring.

"Substitution" or "substituted" refers to substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, a bridged ring group, a spiro ring group, a fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$, $-(CH_2)_m$-alkynyl-$R^a$ (wherein m and n are 0, 1 or 2), arylthio, thiocarbonyl, silyl, $-NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulphonyl, or trifluoromethylsulphonyl; alternatively, $R^b$ and $R^c$ may form five- or six-membered cycloalkyl or heterocyclyl; $R^a$ and $R^d$ are each independently selected from aryl, heteroaryl, alkyl, alkoxy, cycloalkyl, heterocyclyl, carbonyl, an ester group, a bridged ring group, a spiro ring group or a fused ring group.

"Substituted with 1 to X substituents selected from ..." refers to a substitution with 1, 2, 3 ... X substituents selected from ..., wherein X is selected from any integer between 1 and 10. For example, "substituted with 1 to 4 $R^k$" refers to a substitution with 1, 2, 3 or 4 $R^k$. For example, "substituted with 1 to 5 substituents selected from ..." refers to a substitution with 1, 2, 3, 4 or 5 substituents selected from ... For example, "bridged-heterocyclic ring is optionally substituted with 1 to 4 substituents selected from H or F" means that the bridged-heterocyclic ring is optionally substituted with 1, 2, 3 or 4 substituents selected from H or F.

An X- to Y-membered ring (X and Y are integers, $3 \leq X < Y$, and $X < Y \leq 20$ (i.e., any integer from 4 to 20)) includes X-, X+1-, X+2-, X+3-, X+4-,...or Y-membered rings. Rings include heterocycle, carbocycle, an aromatic ring, aryl, heteroaryl, cycloalkyl, a mono-heterocyclic ring, a fused-heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

A $C_{x-y}$ carbocyclic ring (including aryl, cycloalkyl, a mono-carbocyclic ring, a spiro-carbocyclic ring, a fused-carbocyclic ring, or a bridged-carbocyclic ring) includes $C_x$-, $C_{x+1}$-, $C_{x+2}$-, $C_{x+3}$-, $C_{x+4}$-,...or $C_y$-membered rings (x is an integer, $3 \leq x < y$, and y is any integer from 4 to 20). For example, "$C_{3-6}$ cycloalkyl" refers to $C_3$, $C_4$, $C_5$ or $C_6$ cycloalkyl.

When a group has one or more connectable sites, any one or more sites of the group can be connected to other groups via chemical bonds. When the connection mode of the chemical bond is indeterminate and there are hydrogen atoms at the connectable sites, the number of H atoms at the site will decrease correspondingly with the number of connected chemical bonds when the chemical bond is connected, forming a group with the corresponding valence. For example,

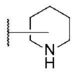

indicates that any connectable site on the piperidyl group can be connected to other groups via one chemical bond, including at least

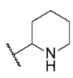, 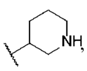, 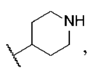, 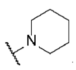

these 4 connection modes. Even if an H atom is shown on the N atom,

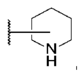,

also includes

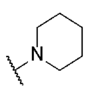.

For example,

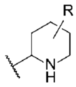

indicates that the R group on the piperidyl group can be located on C or N, including at least

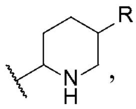, 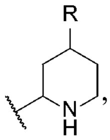, 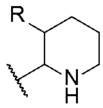, 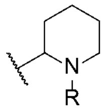, 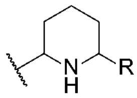.

When the connecting groups listed do not specify their connection direction, their connection directions include both the left-to-right and right-to-left reading orders. For example, for A-L-B, wherein L is -M-W-, it includes both A-M-W-B and A-W-M-B.

Polyacrylic resin is a copolymer formed by polymerizing one or more of the following: methacrylic acid, methyl methacrylate, ethyl methacrylate, butyl methacrylate, trimethylaminoethyl methacrylate chloride, dimethylaminoethyl methacrylate, methyl acrylate, and ethyl acrylate, including but not limited to EUDRAGIT, polyacrylic resin II (copolymer of methacrylic acid and methyl methacrylate (1 : 1)), polyacrylic resin III, polyacrylic resin IV, poly(amino methacrylate) I, poly(amino methacrylate) II, wherein EUDRAGIT includes, but is not limited to methacrylic acid copolymer type A (EUDRAGIT L100), methacrylic acid copolymer type B (EUDRAGIT S100), methacrylic acid copolymer type C (EUDRAGIT 100-55), water dispersion of methacrylic acid-ethyl acrylate copolymer (EUDRAGIT L30D-55), etc.

The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound according to the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated substances, solvates, prodrugs, metabolites, pharmaceutically acceptable salts or eutectic crystals thereof and other chemical components, wherein "other chemical components" refer to pharmaceutically acceptable carriers, excipients and/or one or more other therapeutic agents.

"Carrier" refers to a material that does not cause significant irritation to an organism and does not eliminate the biological activity and characteristics of a compound administered.

The term "excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatine, vegetable oils, polyethylene glycols, diluents, granulating agents, lubricants, binders and disintegrants.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or *in vivo* to obtain a parent compound. When the prodrug of the present invention is administered to a mammalian individual, the prodrug is split to form a free amino or carboxyl group.

The term "eutectic crystal" refers to a crystal formed by the combination of active pharmaceutical ingredient (API) and eutectic crystal former (CCF) under the action of hydrogen bonds or other non-covalent bonds. The pure state of API and CCF are both solid at room temperature, and there is a fixed stoichiometric ratio between various components. The eutectic crystal is a multi-component crystal, which includes both a binary eutectic crystal formed between two neutral solids and a multi-element eutectic crystal formed between a neutral solid and a salt or solvate.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerization and amide-imino alcohol isomerization.

The term "$IC_{50}$" refers to the concentration of a drug or inhibitor required to inhibit half of a given biological process (or a component of the process such as an enzyme, a receptor and a cell).
Unless otherwise specified, the term "content %" of a substance in the pharmaceutical composition of the present application means the weight of the substance as a percentage of the total weight of the pharmaceutical composition.

Detailed Description of Embodiments

The technical solutions of the present invention will be described in detail by the following examples, but the scope of protection of the present invention includes but is not limited thereto.
The compounds used in the reactions described herein are prepared according to organic synthesis techniques known to those skilled in the art, starting from commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.
The structures of the compounds are determined by nuclear magnetic resonance (NMR) or (and) mass spectrometry (MS). The NMR shift (δ) is given in the unit of $10^{-6}$ (ppm). NMR is determined with NMR instruments (Bruker Avance III 400 and Bruker Avance 300); the solvent for determination is deuterated dimethylsulphoxide (DMSO-$d_6$), deuterated chloroform ($CDCl_3$) or deuterated methanol ($CD_3OD$); and the internal standard is tetramethylsilane (TMS).

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai $HSGF_{254}$ or Qingdao $GF_{254}$ silica gel plate is used as a thin layer chromatography silica plate; and the silica gel plate for the thin layer chromatography (TLC) is of the specification of 0.15 mm-0.20 mm, and the specification when separating and purifying a product by thin layer chromatography is 0.4 mm-0.5 mm.

For column chromatography, Yantai Huanghai silica gel of 200-300 mesh silica gel is generally used as a carrier.

**Preparation of intermediate 3**

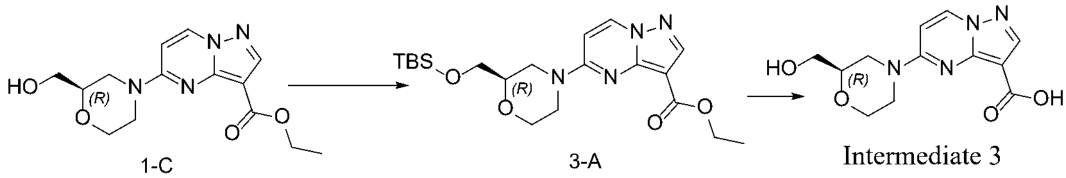

1-C 3-A Intermediate 3

Step 1: Preparation of 3-A

1-C (2.3 g, 7.51 mmol) was dissolved in 20 mL of dichloromethane, and triethylamine (2.28 g, 22.53 mmol) was added. The mixture was cooled to 0°C, and TBSCl (1.70 g, 11.28 mmol) was added. The mixture was reacted at room temperature for 16 h. The reaction system was concentrated under reduced pressure, and the crude was separated and purified by silica gel column chromatography (petroleum ether/ethyl acetate (v/v) = 65 : 35) to obtain 3-A (1.4 g, yield: 44%).

Step 2: Preparation of intermediate 3

3-A (1.4 g, 3.33 mmol) was dissolved in a mixed solvent of tetrahydrofuran/water (v/v) = 4 : 1, lithium hydroxide monohydrate (699 mg, 16.66 mmol) was added, and the mixture was reacted at room temperature for 16 h. The reaction system was adjusted to pH 5 with 0.5 mol/L aqueous hydrochloric acid solution, extracted with a mixed solvent of dichloromethane/methanol (v/v) = 10 : 1 (50 mL × 3), and the organic phase was washed with saturated brine (30 mL × 3), dried over anhydrous sodium sulphate, and concentrated under reduced pressure to obtain a crude intermediate 3 (0.57 g).

**Preparation of intermediate 5**

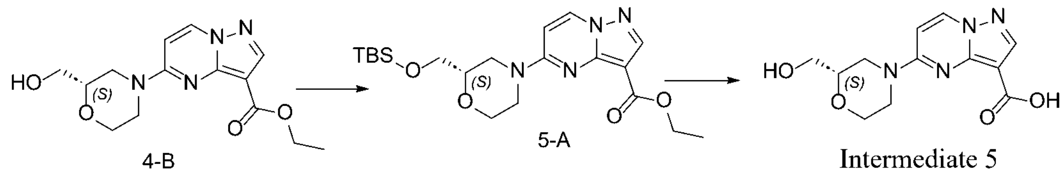

4-B 5-A Intermediate 5

Step 1: Preparation of 5-A

4-B (4 g, 13.06 mmol) and triethylamine (5.29 g, 52.28 mmol) were dissolved in 50 mL of dichloromethane, TBSCl (3.94 g, 26.14 mmol) was added, and the mixture was reacted at room temperature for 16 h. 50 mL of water was added to the reaction system, and liquid separation was carried out. The organic phase was dried over anhydrous sodium sulphate, and concentrated under reduced pressure. The crude was separated and purified by silica gel column chromatography (petroleum ether/ethyl acetate (v/v) = 65 : 35) to obtain 5-A (2 g, yield: 36%).

Step 2: Preparation of intermediate 5

5-A (1.8 g, 4.28 mmol) and lithium hydroxide (0.32 g, 13.36 mmol) were added into a 100 mL single-necked flask, and 20 mL of methanol and 10 mL of water were added, and the mixture was reacted at 60°C for 4 h. The reaction system was cooled to room temperature, and 50 mL of water was added. The pH was adjusted to 2 with 1 mol/L aqueous hydrochloric acid solution. The mixture was extracted with ethyl acetate (50 mL × 3). The organic phase was dried over anhydrous sodium sulphate and concentrated under reduced pressure to obtain a crude intermediate 5 (2 g).

**Preparation of the hydrochloride salt of intermediate 6 (trans)**

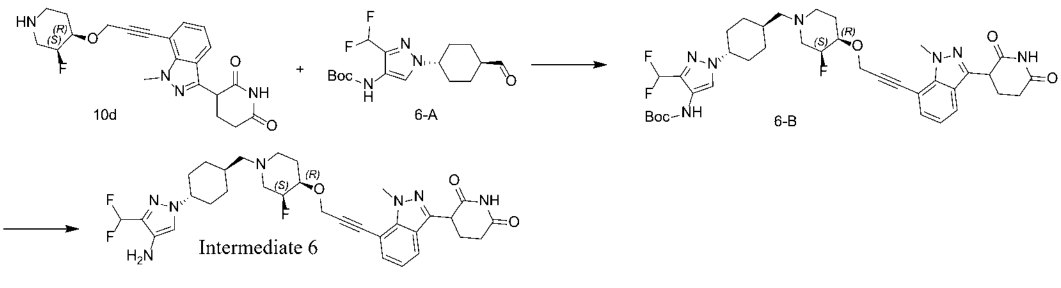

10d 6-A 6-B Intermediate 6

Step 1: Preparation of 6-B

10d was prepared with reference to the synthesis method of 12d. The trifluoroacetate salt of the crude 10d (5.57 g) was dissolved in 40 mL DMA, and sodium bicarbonate (1.49 g, 17.74 mmol) was added. After stirring at room temperature for 15 min, 6-A (3.66 g, 10.66 mmol) (synthesis method, see WO 2021158634), 1.2 mL acetic acid and 4 Å molecular sieves (5 g) were added. After stirring at room temperature for 2 h, sodium triacetoxyborohydride (3.77 g, 17.79 mmol) was added and reacted at room temperature for 16 h. 150 mL of aqueous saturated sodium bicarbonate solution was added to the reaction system, extracted with 100 mL of dichloromethane, the organic phase was dried over anhydrous sodium sulphate, concentrated under reduced pressure, and the crude was separated and purified by silica gel column chromatography (dichloromethane/ethyl acetate (v/v) = 1 : 1) to obtain 6-B (3.1 g, yield: 40%).

Step 2: Synthesis of the hydrochloride salt of intermediate 6

6-B (600 mg, 0.83 mmol) was dissolved in 10 mL of 1,4-dioxane, and 10 mL of 4 mol/L hydrogen chloride 1,4-dioxane solution was added. The mixture was reacted at room temperature for 2 h. The reaction system was concentrated under reduced pressure to obtain the hydrochloride salt of a crude intermediate 6 (0.65 g).
LCMS m/z = 626.3 $[M+1]^+$.

**Preparation of intermediate 7 (trans)**

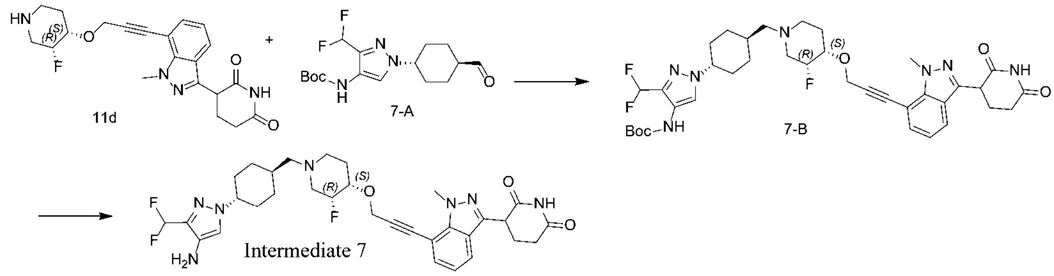

11d 7-A 7-B Intermediate 7

Step 1: Preparation of 7-B

11d was prepared with reference to the synthesis method of 12d. The trifluoroacetate salt of the crude 11d (5.0 g) was dissolved in 60 mL DMA, and sodium bicarbonate (1.68 g, 20.0 mmol) was added. After stirring at room temperature for 15 min, 7-A (3.29 g, 9.58 mmol) (synthesis method, see WO 2021158634), 1 mL acetic acid and 4 Å molecular sieves (5 g) were added. After stirring at room temperature for 2 h, sodium triacetoxyborohydride (3.38 g, 15.95 mmol) was added and reacted at room temperature for 16 h. 150 mL of aqueous saturated sodium bicarbonate solution was added to the reaction system, extracted with 100 mL of dichloromethane, the organic phase was dried over anhydrous sodium sulphate, concentrated under reduced pressure, and the crude was separated and purified by silica gel column chromatography (dichloromethane/ethyl acetate (v/v) = 1 : 1) to obtain 7-B (1.0 g, yield: 14%).

Step 2: Preparation of intermediate 7

7-B (1.1 g, 1.52 mmol) was added to 30 mL of acetonitrile, and p-toluenesulphonic acid monohydrate (0.86 g, 4.52 mmol) was added, and the mixture was reacted at room temperature for 3 h. The reaction solution was concentrated under reduced pressure, 100 mL of ethyl acetate was added, and the pH was adjusted to 9 with saturated sodium bicarbonate solution. The organic phase was separated, dried over anhydrous sodium sulphate, and concentrated under reduced pressure to obtain a crude intermediate 7 (0.9 g).

**Synthesis example 12: Preparation of compound 12 (Trans)**

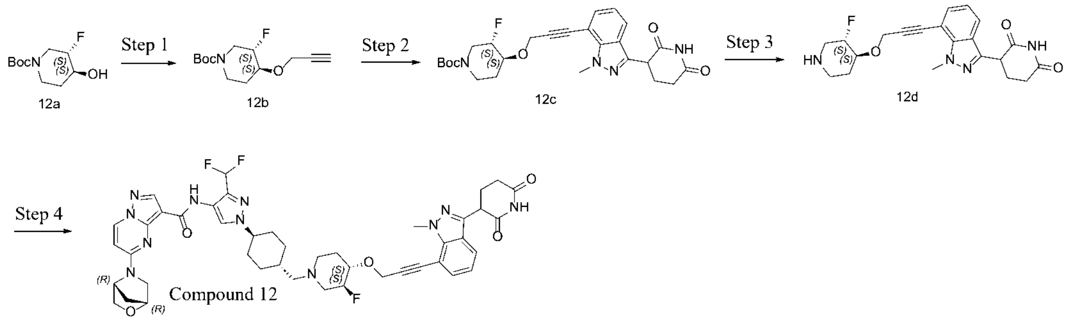


12a 12b 12c 12d Compound 12

Step 1: Preparation of 12b

12a (1.3 g, 5.93 mmol) was added to 50 mL of tetrahydrofuran, and the mixture was cooled to 0°C. 0.22 g of sodium hydride was added, and the mixture was stirred at 0°C for 30 min. Then, 3-bromoprop-1-yne (0.77 g, 6.48 mmol) was added and the mixture was reacted at room temperature for 12 h. 100 mL of saturated ammonium chloride solution was added to the reaction system, and the mixture was extracted with 100 mL of ethyl acetate. The organic phase was dried over anhydrous sodium sulphate and concentrated under reduced pressure. The crude was separated and purified by silica gel column chromatography (petroleum ether/ethyl acetate (v/v) = 4 : 1) to obtain 12b (1.1 g, yield: 72%).

Step 2: Preparation of 12c

12b (0.07 g, 0.27 mmol) and 3-(7-iodo-1-methyl-1H-indazol-3-yl)piperidine-2,6-dione (2C) (0.1 g, 0.27 mmol) were added to 5 mL of DMF, and 1 mL of triethylamine, CuI (0.02 g, 0.1 mmol) and $PdCl_2(PPh_3)_2$ (0.035 g, 0.05 mmol) were added. The mixture was subjected to nitrogen replacement three times, and reacted at 60°C for 4 h under nitrogen protection. The reaction liquid was cooled to room temperature, 30 mL of ethyl acetate was added, and the organic phase was washed with 50 mL of purified water. The mixture was dried over anhydrous sodium sulphate, and concentrated under reduced pressure. The crude was separated and purified by silica gel column chromatography (petroleum ether/ethyl acetate (v/v) = 2 : 1-1 : 1) to obtain 12c (0.09 g, yield: 67%).

Step 3: Preparation of the trifluoroacetate salt of 12d

Compound 12c (0.09 g, 0.18 mmol) was added to 3 mL of dichloromethane, and 1 mL of trifluoroacetic acid was added, and the mixture was reacted at room temperature for 2 h. The reaction liquid was concentrated under reduced pressure to obtain the trifluoroacetate salt of a crude 12d (0.1 g).
LCMS m/z = 399.1 $[M+1]^+$.

Step 4: Preparation of compound 12

To the above-mentioned trifluoroacetate salt (0.1 g) of the crude 12d, 2 mL of triethylamine and 5 mL of DMA were added to dissolve same. Then, 6 h (0.087 g, 0.18 mmol) was added, and after stirring at room temperature for 3 h, sodium triacetoxyborohydride (0.042 g, 0.2 mmol) was added, and the mixture was reacted at room temperature for 16 h. The reaction liquid was filtered, and the filtrate was concentrated under reduced pressure. The obtained crude was first subjected to chiral preparation and then conventional preparation, and lyophilized to obtain compound 12 (22 mg, yield: 14%).
Chiral preparation method:

instrument and preparative column: Waters 150 SFC preparative liquid chromatography, preparative column model Chiralpak Column;
Mobile phase system: $sCO_2$ (supercritical $CO_2$)/a mixed solvent of isopropanol and acetonitrile, isocratic elution: $sCO_2$/a mixed solvent of isopropanol and acetonitrile = 2 : 3; flow rate: 100 mL/min.

Analytical methods for target compounds:
instrument: SHIMADZU LC-30AD sf; chromatographic column: Chiralpak AD-3; Specification: 50 × 4.6 mm I.D., 3 μm; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A : B = 2 : 3; retention time of target compounds: 1.16 min.
Conventional preparation method:
instrument and preparative column: SHIMADZU LC-20AP preparative liquid chromatography, preparative column model Phenomenex C18. Preparation method: the crude was dissolved with acetonitrile and water to prepare a sample liquid. Mobile phase system: acetonitrile/water (containing 0.05% ammonium bicarbonate). Gradient elution method: a gradient from 45% acetonitrile to 75% acetonitrile (elution time 15 min).
$^1H$ NMR (400 MHz, DMSO-$d_6$) δ 10.90 (s, 1H), 9.56 - 9.42 (m, 1H), 8.77 (d, 1H), 8.42 - 8.35 (m, 1H), 8.28 - 8.22 (m,1H), 7.79 (d, 1H), 7.52 (d, 1H), 7.27 - 6.94 (m, 2H), 6.89 - 6.42 (m, 1H), 5.33 - 5.01 (m, 1H), 4.85 - 4.70 (m, 1H), 4.63 (s, 2H), 4.59 - 4.35 (m, 2H), 4.29 (s, 3H), 4.24 - 4.08 (m, 1H), 3.88 - 3.40 (m, 5H), 3.10 - 2.95 (m, 1H), 2.78 - 2.55 (m, 3H), 2.45 - 1.37 (m, 17H), 1.11 - 0.95 (m, 2H).
LCMS m/z = 434.8 $[M/2+1]^+$.

**Synthesis example 19: Preparation of compound 19 (Trans)**

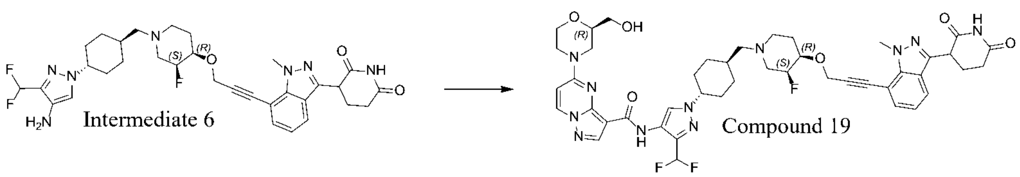

The hydrochloride salt of the above-mentioned crude intermediate 6 (130 mg) was dissolved in 5 mL of acetonitrile, and the above-mentioned crude intermediate 3 (53 mg) and TCFH (67 mg, 0.24 mmol) were added, and NMI (0.11 g, 1.34 mmol) was added, and the mixture was reacted at room temperature for 16 h. The reaction system was concentrated under reduced pressure, 10 mL of water was added to the residue, and the mixture was filtered. The filter cake was washed with 5 mL of water, dissolved in 10 mL of DCM, dried over anhydrous sodium sulphate, and concentrated under reduced pressure. The crude was separated and purified by silica gel column chromatography (dichloromethane/methanol (v/v) = 12 : 1). The obtained crude was subjected to chiral preparative chromatography to obtain chiral isomer 1 (18.2 mg, two-step yield from compound 3-A: 7%) and chiral isomer 2 (12.5 mg, two-step yield from compound 3-A: 5%) of compound 19, respectively.

Chiral preparation method:
instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm; mobile phase A: acetonitrile; mobile phase B: methanol; column temperature: 35°C; flow rate: 1 mL/min; wavelength: 220 nm; Elution program: Isocratic elution: mobile phase A : B = 3 : 7;
retention time of chiral isomer 1: 5.313 min.

$^1$H NMR (400 MHz, DMSO-$d_6$) δ 10.90 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H).

LCMS m/z = 886.4 $[M+1]^+$.
retention time of chiral isomer 2: 6.457 min.

$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.60 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H).

LCMS m/z = 886.4 $[M+1]^+$.

The obtained chiral isomer 2 was subjected to acidic preparative chromatography to obtain the trifluoroacetate salt of chiral isomer 2.

Acidic preparation method:
instrument and preparative column: Shimadzu LC-20AP preparative liquid chromatography, preparative column model Welch Xtimate C18; Mobile phase system: water (containing 0.1% trifluoroacetic acid)/acetonitrile, gradient elution: water (containing 0.1% trifluoroacetic acid)/acetonitrile = 18 : 82-48 : 52; flow rate: 25 mL/min.

NMR data of trifluoroacetate salt of chiral isomer 2:
$^1$H NMR (400 MHz, DMSO-$d_6$) δ 10.91 (s, 1H), 9.35 (s, 1H), 8.82 (d, 1H), 8.38 (s, 1H), 8.29 (s, 1H), 7.81 (d, 1H), 7.53 (d, 1H), 7.27 - 7.07 (m, 2H), 6.91 (d, 1H), 4.90 - 4.56 (m, 3H), 4.53 - 3.82 (m, 10H), 3.65 - 3.46 (m, 4H), 3.20 - 3.08 (m, 2H), 3.07 - 2.90 (m, 2H), 2.76 - 2.56 (m, 2H), 2.45 - 1.68 (m, 14H), 1.60 - 1.46 (m, 1H), 1.34 - 1.10 (m, 2H).

**Synthesis example 20: Preparation of compound 20 (Trans)**

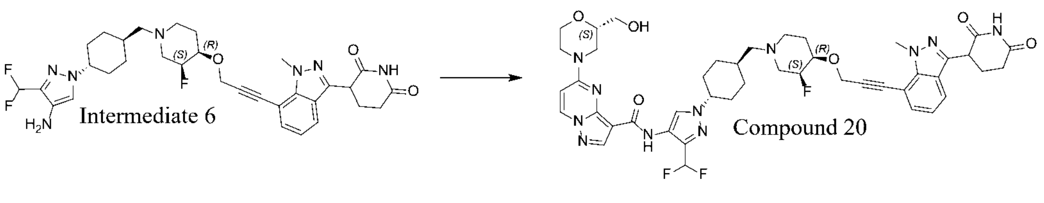


The hydrochloride salt of the above-mentioned crude intermediate 6 (130 mg) was dissolved in 5 mL of acetonitrile, and the above-mentioned crude intermediate 5 (53 mg) and TCFH (67 mg, 0.24 mmol) were added, and NMI (0.11 g, 1.34 mmol) was added, and the mixture was reacted at room temperature for 16 h. The reaction system was concentrated under reduced pressure, 10 mL of water was added to the residue, and the mixture was filtered. The filter cake was washed with 5 mL of water, dissolved in 10 mL of DCM, dried over anhydrous sodium sulphate, and concentrated

under reduced pressure. The crude was separated and purified by silica gel column chromatography (dichloromethane/methanol (v/v) = 12 : 1). The obtained crude was subjected to chiral preparative chromatography to obtain chiral isomer 1 (10.3 mg, two-step yield from compound 5-A: 4%) and chiral isomer 2 (9.8 mg, two-step yield from compound 5-A: 4%) of compound 20, respectively.

Chiral preparation method:
instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm; mobile phase A: acetonitrile; mobile phase B: methanol; column temperature: 35°C; flow rate: 1 mL/min; wavelength: 220 nm; Elution program: Isocratic elution: mobile phase A : B = 3 : 7;
retention time of chiral isomer 1: 5.229 min.

$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.89 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).

LCMS m/z = 886.4 $[M+1]^+$.
retention time of chiral isomer 2: 6.378 min.

$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.60 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).

LCMS m/z = 886.4 $[M+1]^+$.

**Synthesis example 24: Preparation of compound 24 (Trans)**

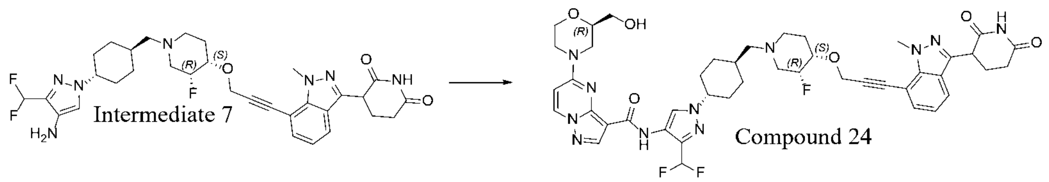


The above-mentioned crude intermediate 7 (0.12 g) was added to 5 mL of acetonitrile, and the above-mentioned crude intermediate 3 (0.07 g) and TCFH (0.084 g, 0.3 mmol) were added, and NMI (0.08 g, 0.97 mmol) was added at 0°C, and the mixture was reacted at room temperature for 16 h. The reaction system was concentrated under reduced pressure, and the residue was subjected to chiral preparative chromatography to obtain chiral isomer 1 (30.5 mg) and chiral isomer 2 (28 mg) of compound 24, respectively.

Chiral preparation method:
instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm; mobile phase A: acetonitrile; mobile phase B: methanol; column temperature: 35°C; flow rate: 1 mL/min; wavelength: 220 nm; Elution program: Isocratic elution: mobile phase A : B = 3 : 7;
retention time of chiral isomer 1: 5.434 min.

$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.89 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H).

LCMS m/z = 886.4 $[M+1]^+$.

retention time of chiral isomer 2: 6.706 min.

$^1H$ NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H).

LCMS m/z = 886.4 $[M+1]^+$.

**Synthesis example 25: Preparation of compound 25 (Trans)**

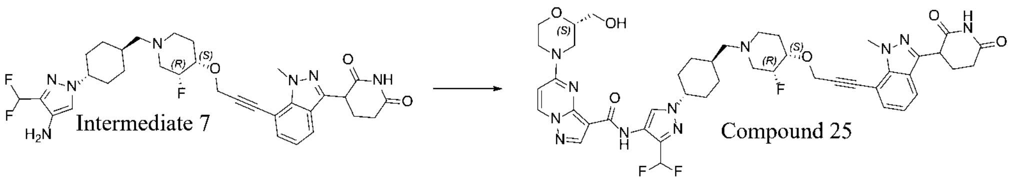


The above-mentioned crude intermediate 7 (0.12 g) was added to 5 mL of acetonitrile, and the above-mentioned crude intermediate 5 (0.07 g) and TCFH (0.084 g, 0.3 mmol) were added, and NMI (0.08 g, 0.97 mmol) was added at 0°C, and the mixture was reacted at room temperature for 16 h. The reaction system was concentrated under reduced pressure, and the residue was subjected to chiral preparative chromatography to obtain chiral isomer 1 (25 mg) and chiral isomer 2 (26.4 mg) of compound 25, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm; mobile phase A: acetonitrile; mobile phase B: methanol; column temperature: 35°C; flow rate: 1 mL/min; wavelength: 220 nm; Elution program: Isocratic elution: mobile phase A : B = 3 : 7;

retention time of chiral isomer 1: 5.364 min.

$^1H$ NMR (400 MHz, DMSO-$d_6$) δ 10.89 (s, 1H), 9.34 (s, 1H), 8.81 (d, 1H), 8.36 (s, 1H), 8.28 (s, 1H), 7.79 (d, 1H), 7.52 (d, 1H), 7.27 - 6.94 (m, 2H), 6.90 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).

LCMS m/z = 886.4 $[M+1]^+$.

retention time of chiral isomer 2: 6.619 min.

$^1H$ NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.60 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).

LCMS m/z = 886.4 $[M+1]^+$.

The compounds in Table S-2 below were prepared with reference to the preparation method of synthesis example 19.

Analytical methods for target compounds:

instrument: Shimadzu LC-20AB with PDA detector; chromatographic column: Chiralpak IE-3; Specification: 150 × 4.6 mm I.D., 3 μm; mobile phase A: acetonitrile; mobile phase B: methanol; column temperature: 35°C; flow rate: 1 mL/min; wavelength: 220 nm; Elution program: Isocratic elution: mobile phase A : B = 3:7.

Table S-2

| Compound No. | Compound structure | Chiral isomer 1 retention time and LCMS m/z | chiral isomer 2 retention time and LCMS m/z |
|---|---|---|---|
| 17 | 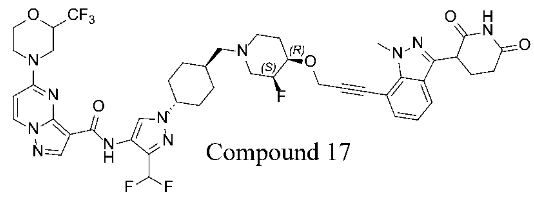 Compound 17 | 3.368 min; 924.4 $[M+1]^+$ | 4.029 min; 924.4 $[M+1]^+$ |
| 18 | 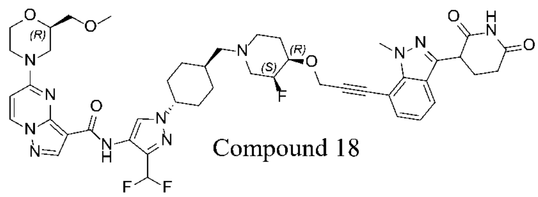 Compound 18 | 6.469 min; 900.5 $[M+1]^+$ | 7.967 min; 900.4 $[M+1]^+$ |
| 21 | 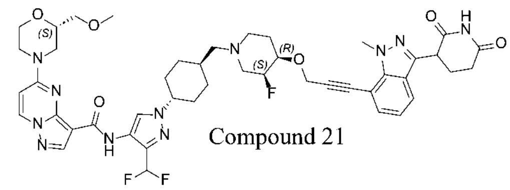 Compound 21 | 6.563 min; 900.4 $[M+1]^+$ | 8.07 min; 900.4 $[M+1]^+$ |
| 22 | 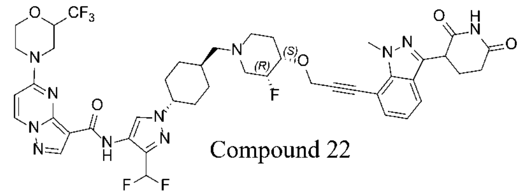 Compound 22 | 3.449 min; 924.4 $[M+1]^+$ | 4.165 min 924.4 $[M+1]^+$ |
| 23 | 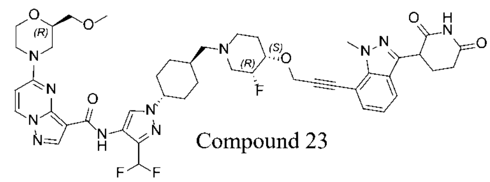 Compound 23 | 6.645 min; 900.5 $[M+1]^+$ | 8.228 min; 900.4 $[M+1]^+$ |
| 26 | 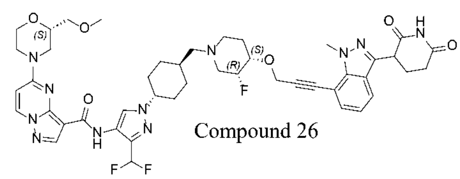 Compound 26 | 6.733 min; 900.4 $[M+1]^+$ | 8.385 min; 900.5 $[M+1]^+$ |

**Formulation examples:**

Unless otherwise specified, a proportion (%) refers to the weight of a component as a percentage of the total weight of the prescription, and a single dose refers to the formulation strength.

SLS: Sodium lauryl sulphate; TPGS: Vitamin E polyethylene glycol succinate;
HPβ CD: Hydroxypropyl-β-cyclodextrin;
HPMCP: Hydroxypropyl methylcellulose phthalate; HPMCAS: Hydroxypropyl methylcellulose acetate succinate;
HPMC: Hydroxypropyl methylcellulose; $CaHPO_4$: Dicalcium phosphate.

Examples 1-3 A capsule was prepared with compound 20 by adding a solubilizer.

| No. | Example 1 | Example 2 | Example 3 |
|---|---|---|---|
| Prescription composition | Single dose (mg) | Single dose (mg) | Single dose (mg) |
| Compound 20 | 10.0 | 20.0 | 100.0 |
| SLS | 160.0 | 320.0 | 400.0 |
| HPMC E5 | / | / | 8.0 |
| CaHPO4 | / | / | 80.0 |
| Lactose | 201.3 | 402.5 | / |
| Magnesium stearate | 3.8 | 7.5 | 6.0 |
| Total | 375.0 | 750.0 | 600.0 |
| Formulation | Capsule | Capsule | Capsule |
| Preparation process | The components were mixed well and then directly filled into capsules | The components were mixed well and then directly filled into capsules | Compound 20 and non-active ingredients (except magnesium stearate) were mixed well, an appropriate amount of 75% ethanol was added for wet granulation with a 24-mesh sieve. The wet granules were dried in an oven at 60°C and then sized using a 24-mesh sieve. Magnesium stearate was added and the resulting mixture was mixed well and filled into capsules |

Examples 4-8 A **solid dispersion** was prepared with compound 19 and different polymers.

| Example | | Example 4 | Example 5 | Example 6 |
|---|---|---|---|---|
| Prescription composition | | Single dose (mg) | Single dose (mg) | Single dose (mg) |
| Formulation intermediate | Compound 19 | 25.0 | 50.0 | 50.0 |
| | HPMCP | 75.0 | / | 150.0 |
| | Eudragit L100 | / | 150.0 | / |
| Lactose | | 90.5 | 181.0 | 181.0 |
| Microcrystalline cellulose | | 45.0 | 90.0 | 90.0 |
| Povidone | | 8.0 | 16.0 | 16.0 |
| Crospovidone | | 8.0 | 16.0 | 16.0 |
| Croscarmellose sodium | | 8.0 | 16.0 | 16.0 |
| Magnesium stearate | | 2.5 | 5.0 | 5.0 |
| Total | | 262.0 | 524.0 | 524.0 |
| Formulation | | Tablet | Tablet | Tablet |

(continued)

| Example | Example 4 | Example 5 | Example 6 |
|---|---|---|---|
| Formulation process | Compound 19 and HPMCP or Eudragit L100 at the amounts according to the prescription were completely dissolved in an appropriate organic solvent (for example, dichloromethane : methanol =2 : 1 (v/v)), and the resulting mixture was subjected to spray drying to obtain a dry powder, i.e., **the formulation intermediate containing compound 19.** The formulation intermediate and other components at the amounts according to the prescription were mixed well and an appropriate amount of purified water was added for wet granulation with a 24-mesh sieve. The wet granules were dried in an oven at 60°C and then sized using a 24-mesh sieve. Magnesium stearate was added and the resulting mixture was mixed and compressed into tablets | | |

| Example | | Example 7 | Example 8 |
|---|---|---|---|
| Prescription composition | | Single dose (mg) | Single dose (mg) |
| Formulation intermediate | Compound 19 | 50.0 | 100 |
| | Eudragit L100 | 150.0 | 200 |
| Lactose | | 181.0 | 362.0 |
| Microcrystalline cellulose | | 90.0 | 180.0 |
| Crospovidone | | 16.0 | 32.0 |
| Croscarmellose sodium | | 16.0 | 32.0 |
| Magnesium stearate | | 5.0 | / |
| Colloidal silicon dioxide | | / | 10.0 |
| Total | | 508.0 | 1016.0 |
| Formulation | | Capsule | Granule |
| Formulation process | | The preparation method of the formulation intermediate was the same as in Example 4. After the formulation intermediate and other components at the amounts according to the prescription were mixed well, the resulting mixture was filled into capsules | The preparation method of the formulation intermediate was the same as in Example 4. After the formulation intermediate and other components (except colloidal silicon dioxide) at the amounts according to the prescription were mixed well, the resulting mixture was subjected to dry granulation. Colloidal silicon dioxide was then added and the resulting mixture was mixed and packaged |

Examples 9-13 A **solid dispersion** was prepared with compound 24, polymers and solubilizer.

| Example | | Example 9 | Example 10 | Example 11 |
|---|---|---|---|---|
| Prescription composition | | Single dose (mg) | Single dose (mg) | Single dose (mg) |
| Formulation intermediate | Compound 24 | 25.0 | 100.0 | 100.0 |
| | HPMCAS | 50.0 | 200.0 | 200.0 |
| | TPGS | 8.3 | 33.2 | 33.2 |

(continued)

| Example | Example 9 | Example 10 | Example 11 |
|---|---|---|---|
| Microcrystalline cellulose | 66.7 | 266.8 | 266.8 |
| Crospovidone | 10.0 | 40.0 | 40.0 |
| Croscarmellose sodium | 10.0 | 40.0 | 40.0 |
| Magnesium stearate | 2.0 | 8.0 | 8.0 |
| Total | 172.0 | 688.0 | 688.0 |
| Formulation | Tablet | Tablet | Capsule |
| Formulation process | Compound 24, HPMCAS and TPGS at the amounts according to the prescription were completely dissolved in an appropriate organic solvent (for example, dichloromethane : methanol =2 : 1 (v/v)), and the resulting mixture was subjected to spray drying to obtain a dry powder, i.e., the formulation intermediate containing compound 24. After the formulation intermediate and other components at the amounts according to the prescription were mixed well, the resulting mixture was compressed into tablets | | The preparation method of the formulation intermediate was the same as in Example 9. After the formulation intermediate and other components at the amounts according to the prescription were mixed well, the resulting mixture was filled into capsules |

| Example | | Example 12 | Example 13 |
|---|---|---|---|
| Prescription composition | | Single dose (mg) | Single dose (mg) |
| Formulation intermediate | Compound 25 | 200.0 | 200.0 |
| | HPMCAS | 400.0 | 400.0 |
| | TPGS | 66.4 | 66.0 |
| Microcrystalline cellulose | | 533.6 | 654.9 |
| Xanthan gum | | / | 41.3 |
| Crospovidone | | 80.0 | / |
| Croscarmellose sodium | | 80.0 | / |
| Colloidal silicon dioxide | | 16.0 | 13.8 |
| Total | | 1376.0 | 1376.0 |
| Formulation | | Granule | Dry suspension |
| Formulation process | | The preparation method of the formulation intermediate was the same as in Example 9. After the formulation intermediate and other components (except colloidal silicon dioxide) at the amounts according to the prescription were mixed well, the resulting mixture was subjected to dry granulation. Colloidal silicon dioxide was then added and the resulting mixture was mixed and packaged | The preparation method of the formulation intermediate was the same as in Example 9. After the formulation intermediate and other components at the amounts according to the prescription were mixed well, the resulting mixture was packaged |

| Example | Example 14 |
|---|---|
| Prescription composition | Single dose (mg) |

(continued)

| Example | | Example 14 |
|---|---|---|
| Formulation intermediate | Compound 20 | 25.0 |
| | HPβ CD | 100.0 |
| Mannitol | | 15.0 |
| Microcrystalline cellulose | | 15.0 |
| Croscarmellose sodium | | 24.0 |
| Colloidal silicon dioxide | | 3.0 |
| Sodium stearyl fumarate | | 3.0 |
| Total | | 185.0 |
| Formulation | | Capsule |
| Formulation process | | Compound 20 and HPβ CD at the amounts according to the prescription were completely dissolved in an appropriate organic solvent (for example, dichloromethane : methanol =3 : 1 (w/w)), and the resulting mixture was subjected to spray drying to obtain a dry powder, i.e., the formulation intermediate containing compound 20. After the formulation intermediate and other components at the amounts according to the prescription were subjected to dry granulation, the resulting granules were filled into capsules |

| Example | Example 15 |
|---|---|
| Prescription composition | Single dose (mg) |
| Compound 20 | 25 |
| SLS | 100 |
| Total | 125 |
| Formulation | Capsule |
| Formulation process | After compound 20 and non-active ingredients were mixed well, the resulting mixture was filled into capsules |

**Biological test examples**

**Test example 1: Study on IRAK4 degradation activity in hPBMC cells (24 hours)**

hPBMC cells are human peripheral blood mononuclear cells. Peripheral venous blood was taken from healthy volunteers, and hPBMCs were isolated using Ficoll density gradient centrifugation (Ficoll-PaqueTM PLUS 1.077, GE, Cat. 17-1140-02). Culture conditions: RPMI-1640 + 10% FBS + 1% penicillin-streptomycin solution. The cells were cultured in an incubator at 37°C and 5% $CO_2$. The cells were plated in a 24-well plate at $1 \times 10^6$ cells/well. After plating, compounds at different concentrations were added, and the cells were cultured in an incubator at 37°C and 5% $CO_2$ for 24 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, the protein was quantified using a BCA kit (Beyotime, Cat. P0009), and then the protein was diluted to 1 mg/mL. The expression of IRAK4 (CST, Cat. 4363S) and the internal reference cofilin (CST, Cat. 5175S) were detected using a fully automated western blot quantitative analyser (Proteinsimple). The expression level of IRAK4 relative to the internal reference was calculated using compass software. The remaining IRAK4 protein IRAK4% relative to the vehicle control group at different doses was calculated using formula (1), and the IRAK4 protein degradation relative to the vehicle control group at different doses was calculated using formula (2), where $IRAK4_{administration}$ was the expression level of IRAK4 in the administration groups at different doses, and $IRAK4_{vehicle}$ was the expression level of IRAK4 in the vehicle control group. An IRAK4 degradation-drug concentration curve was fit using Graphpad Prism 8 software, and the $DC_{50}$ was calculated.

$$IRAK4\% = IRAK4_{administration} / IRAK4_{vehicle} \times 100\% \qquad \text{formula 1}$$

$$IRAK4\ degradation\% = 100\% - IRAK4\% \qquad \text{formula 2}$$

Table 1 $DC_{50}$ of test compounds for IRAK4 protein degradation in hPBMC cells

| Compound No. | $DC_{50}$ (nM) |
|---|---|
| Chiral isomer 1 of compound 19 | < 20 |
| Chiral isomer 2 of compound 19 | < 20 |
| Chiral isomer 1 of compound 20 | < 20 |
| Chiral isomer 2 of compound 20 | < 20 |
| Chiral isomer 2 of compound 23 | < 20 |
| Chiral isomer 1 of compound 24 | < 20 |
| Chiral isomer 1 of compound 25 | < 20 |
| Chiral isomer 2 of compound 25 | < 20 |

Conclusion: The compounds of the present invention, such as the example compounds, have a given degradation effect on IRAK4 protein in hPBMC cells at 24 h.

**Test example 2: Pharmacokinetic test in mice**

**Experimental animals:** Male ICR mice, about 25 g, 6 mice/compound, purchased from Chengdu Ddossy Experimental Animals Co., Ltd.

**Test design:** On the day of the experiment, 6 ICR mice were randomly grouped according to their body weights. The animals were fasted with water available for 12 to 14 h one day before the administration, and were fed 4 h after the administration.

Table 2.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 2.5 | 0.5 | 5 | Plasma | Intrave nous administration |
| G2 | 3 | The compound of the present invention or control compound | 10 | 1 | 10 | Plasma | Intragastric administration |
| Vehicle for intravenous administration: 5% DMA + 5% Solutol + 90% Saline;<br>Vehicle for intragastric administration: 5% DMSO + 30% PEG400 + 65% (20% SBE-CD);<br>(DMSO: dimethyl sulphoxide; DMA: dimethylacetamide; Solutol: polyethylene glycol-15-hydroxystearate; PEG400: polyethylene glycol 400; SBE-β-CD: sulphobutyl-β-cyclodextrin; Saline: physiological saline;) | | | | | | | |

Before and after the administration, 0.15 mL of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous administration group and intragastric administration group were 0 min, 5 min, 15 min, 30 min, 1 h, 2 h, 4 h, 7 h and 24 h. Before analysis and detection, all samples were stored at -60°C. The samples were analysed quantitatively by LC-MS/MS.

Table 2.2 Pharmacokinetic parameters of compounds of the present invention in plasma of mice

| Test compound | Mode of administration* | $AUC_{0-t}$ (ng/mL·h) |
|---|---|---|
| Chiral isomer 1 of compound 19 | i.g. (10 mg/kg) | 13407 ± 4034 |
| Trifluoroacetate salt of chiral isomer | i.g. (10 mg/kg) | 8304 ± 2852 |
| 2 of compound 19 | | |
| Control compound 1 | i.g. (10 mg/kg) | 2322 ± 146 |
| *Notes: i.g. (intragastrical) administration of compound; | | |

Conclusion: The compounds of the present invention, such as the example compounds, have good oral absorption in mice.

**Test example 3. Test of effect on hERG potassium ion channel**

**Experimental platform:** electrophysiological manual patch-clamp system

**Cell line:** Chinese hamster ovary (CHO) cell line stably expressing hERG potassium ion channel

**Experimental method:** In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to + 20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / I\text{o})] \times 100\%$$

where Inhibition% represents the percentage of inhibition of hERG potassium current by the compound, and *I* and *I*o represent the amplitude of hERG potassium current after and before the administration, respectively.

Compound $IC_{50}$ was calculated using GraphPad Prism 5 software by fitting according to the following equation:

$$\text{Y = Bottom + (Top-Bottom)/(1 + 10^((LogIC50-X)*HillSlope))}$$

where X represents the Log value of the tested concentration of the test sample, Y represents the inhibition percentage at the corresponding concentration, and Bottom and Top represent the minimum and maximum inhibition percentage, respectively.

Table 3. $IC_{50}$ values of test compounds for inhibitory effect on hERG potassium channel current

| Compound | $IC_{50}$ (μM) |
|---|---|
| Chiral isomer 1 of compound 19 | > 20 |

(continued)

| Compound | $IC_{50}$ (μM) |
|---|---|
| Trifluoroacetate salt of chiral isomer 2 of compound 19 | > 40 |
| Chiral isomer 1 of compound 20 | > 40 |
| Chiral isomer 2 of compound 20 | > 40 |
| Chiral isomer 2 of compound 23 | > 40 |
| Chiral isomer 1 of compound 24 | > 40 |
| Chiral isomer 1 of compound 25 | > 40 |
| Chiral isomer 2 of compound 25 | > 40 |
| Control compound 1 | 0.6421 |

Conclusion: The compounds of the present invention, such as the example compounds, do not exhibit obvious inhibitory effects on the hERG potassium channel.

The control compound 1 has the structure as follows, and is synthesized with reference to patent WO 2020113233A1.

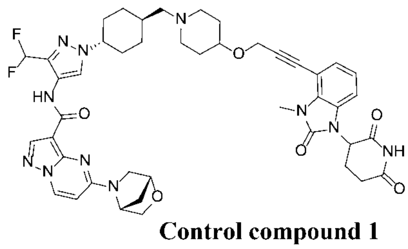

**Control compound 1**

**Test example 4: Pharmacokinetic test in rats**

Experimental animals: Male SD rats, about 200 g, 6-8 weeks old, 6 rats/compound, purchased from Chengdu Ddossy Experimental Animals Co., Ltd.

Test design: on the day of the experiment, the SD rats were randomly grouped according to their body weights. The animals were fasted with water available for 12 to 14 h one day before the administration, and were fed 4 h after the administration.

Table 4.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 2.5 | 0.5 | 5 | Plasma | Intravenous administration |

(continued)

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G2 | 3 | The compound of the present invention or control compound | 10 | 1 | 10 | Plasma | Intragastric administration |
| Vehicle for intravenous administration: 5% DMA + 5% Solutol + 90% Saline;<br>Vehicle for intragastric administration: 5% DMSO + 30% PEG400 + 65% (20% SBE-CD);<br>(DMSO: dimethyl sulphoxide; DMA: dimethylacetamide; Solutol: polyethylene glycol-15-hydroxystearate; PEG400: polyethylene glycol 400; SBE-β-CD: sulphobutyl-β-cyclodextrin; Saline: physiological saline;) | | | | | | | |

Before and after the administration, 0.15 mL of blood was taken from the orbit of the animals under isoflurane anaesthesia and placed in an EDTAK2 centrifuge tube. The blood was centrifuged at 6000 rpm and 4°C for 5 min to collect plasma. The blood collection time points for the intravenous administration group and intragastric administration group were 0 min, 5 min, 15 min, 30 min, 1 h, 2 h, 4 h, 6 h, 8 h and 24 h. Before analysis and detection, all samples were stored at -60°C. The samples were analysed quantitatively by LC-MS/MS.

Conclusion: The compounds of the present invention have good oral absorption in rats.

**Test example 5: Pharmacokinetic test in beagle dogs**

**Experimental animals:** Male beagle dogs, about 8-11 kg, 6 beagle dogs/compound, purchased from Beijing Marshall Biotechnology Co. Ltd.

**Experimental method:** on the day of the experiment, the beagle dogs were randomly grouped according to their body weights. The animals were fasted with water available for 14 to 18 h one day before the administration, and were fed 4 h after the administration.

Table 5.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 1 | 1 | 1 | Plasma | Intravenous administration |
| G2 | 3 | The compound of the present invention or control compound | 5 | 1 | 5 | Plasma | Intragastric administration |
| Vehicle for intravenous administration: 10% DMA + 5% Solutol + 85% Saline;<br>Vehicle for intragastric administration: 5% DMSO + 30% PEG400 + 65% (20% SBE-CD); | | | | | | | |

Before and after the administration, 1 mL of blood was taken from the jugular veins or limb veins, and placed in an EDTAK2 centrifuge tube. The blood was centrifuged at 5000 rpm and 4°C for 10 min to collect plasma. The blood collection

time points for the intravenous administration group and intragastric administration group were 0 min, 5 min, 15 min, 30 min, 1 h, 2 h, 4 h, 6 h, 8 h, 10 h, 12 h and 24 h. Before analysis and detection, all samples were stored at -60°C. The samples were analysed quantitatively by LC-MS/MS.

Conclusion: The compounds of the present invention, such as the example compounds, have good oral absorption in beagle dogs.

**Test example 6: Pharmacokinetic test in monkeys**

**Experimental animals:** Male cynomolgus monkey, about 3-5 kg, 3-6 years old, 6 monkeys/compound, purchased from Suzhou Xishan Biotechnology Inc.

**Experimental method:** on the day of the experiment, the monkeys were randomly grouped according to their body weights. The animals were fasted with water available for 14 to 18 h one day before the administration, and were fed 4 h after the administration.

Table 6.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 1 | 1 | 1 | Plasma | Intravenous administration |
| G2 | 3 | The compound of the present invention or control compound | 5 | 1 | 5 | Plasma | Intragastric administration |
| Vehicle for intravenous administration: 10% DMA + 5% Solutol + 85% Saline;<br>Vehicle for intragastric administration: 5% DMSO + 30% PEG400 + 65% (20% SBE-CD); | | | | | | | |

Before and after the administration, 1 mL of blood was taken from limb veins, and placed in an EDTAK2 centrifuge tube. The blood was centrifuged at 5000 rpm and 4°C for 10 min to collect plasma. The blood collection time points for the intravenous administration group and intragastric administration group were 0 min, 5 min, 15 min, 30 min, 1 h, 2 h, 4 h, 6 h, 8 h, 10 h, 12 h and 24 h. Before analysis and detection, all samples were stored at -60°C. The samples were analysed quantitatively by LC-MS/MS.

Conclusion: The compounds of the present invention, such as the example compounds, have good oral absorption in monkeys.

**Test example 7: Test of stability in liver microsomes**

In this experiment, liver microsomes of five species, including human, monkey, dog, rat and mouse, were used as *in vitro* models to evaluate the metabolic stability of the test compound.

At 37°C, 1 μM test compound was co-incubated with microsomal protein and coenzyme NADPH. At given time points of the reaction (5 min, 10 min, 20 min, 30 min and 60 min), the reaction was terminated by adding ice-cold acetonitrile containing an internal standard. The LC-MS/MS method was used to measure the concentration of the test compound in the sample. $T_{1/2}$ was calculated using the natural logarithm (ln) of the residual rate of the drug in the incubation system and the incubation time. In addition, the intrinsic clearance in liver microsomes $CL_{int(mic)}$ and the intrinsic clearance in liver $CL_{int(Liver)}$ were further calculated.

Conclusion: The compounds of the present invention, such as the example compounds, have good stability in liver microsomes.

**Test example 8: CYP450 enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.

Conclusion: The compounds of the present invention, such as the example compounds, have no obvious inhibitory effect on CYP450 enzyme subtypes.

**Test example 9: Caco2 permeability test**

The experiment used a monolayer of Caco-2 cells incubated in triplicate in a 96-well Transwell plate. A transport buffer solution (HBSS, 10 mM HEPES, pH 7.4 ± 0.05) containing the compound of the present invention (2 μM) or the control compounds of digoxin (10 μM), nadolol (2 μM) and metoprolol (2 μM) was added to the administration end hole on the apical side or the basal side. DMSO-containing transport buffer solution was added to the corresponding receiving end hole. After incubation for 2 hours at 37 ± 1°C, the cell plate was removed and an appropriate amount of a sample was taken from the apical side and basal side and transferred to a new 96-well plate. Acetonitrile containing internal standard was then added to precipitate the protein. Samples were analysed using LC MS/MS and the concentrations of the compounds of the present invention and the control compounds were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was assessed by leakage of Lucifer Yellow.

Conclusion: The compounds of the present invention, such as the example compounds, have good $CaCO_2$ permeability.

**Test example 10: Study on Ikaros degradation activity in hPBMC cells (24 hours)**

hPBMC cells are human peripheral blood mononuclear cells. Peripheral venous blood was taken from healthy volunteers and hPBMCs were isolated using Ficoll density gradient centrifugation (Ficoll-PaqueTM PLUS 1.077, GE, Cat. 17-1140-02). Culture conditions: RPMI-1640 + 10% FBS + 1% penicillin-streptomycin solution. The cells were cultured in an incubator at 37°C and 5% $CO_2$. The cells were plated in a 24-well plate at $1 \times 10^6$ cells/well. After plating, compounds at different concentrations were added, and the cells were cultured in an incubator at 37°C and 5% $CO_2$ for 24 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, and the protein was quantified using a BCA kit (Beyotime, Cat. P0009), and then the protein was diluted to 1 mg/mL. The expression of Ikaros (CST, Cat. # 14859S) and the internal reference β-actin (CST, Cat. # 4970S) were detected using a fully automated western blot quantitative analyser (Proteinsimple). The expression level of Ikaros relative to the internal reference was calculated using compass software. The remaining Ikaros protein relative to the vehicle control group at different doses was calculated using formula (3), and the Ikaros protein degradation relative to the vehicle control group at different doses was calculated using formula (4) to obtain the maximum degradation rate (Dmax) of Ikaros protein by the drug.

$$\text{Remaining Ikaros\%} = \text{Ikaros}_{\text{compound}} / \text{Ikaros}_{\text{vehicle}} \times 100\% \quad \text{formula (3)}$$

$$\text{Ikaros degradation\%} = 100\% - \text{formula (3)} \quad \text{formula (4)}$$

Conclusion: The compounds of the present invention, such as the example compounds, have no obvious degradation effect on Ikaros protein in hPBMC cells.

**Test example 11: Study on Aiolos degradation activity in hPBMC Cells (24 hours)**

hPBMC cells are human peripheral blood mononuclear cells. Peripheral venous blood was taken from healthy volunteers and hPBMCs were isolated using Ficoll density gradient centrifugation (Ficoll-PaqueTM PLUS 1.077, GE, Cat.

17-1140-02). Culture conditions: RPMI-1640 + 10% FBS + 1% penicillin-streptomycin solution. The cells were cultured in an incubator at 37°C and 5% $CO_2$. The cells were plated in a 24-well plate at 1 × $10^6$ cells/well. After plating, compounds at different concentrations were added, and the cells were cultured in an incubator at 37°C and 5% $CO_2$ for 24 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, and the protein was quantified using a BCA kit (Beyotime, Cat. P0009), and then the protein was diluted to 1 mg/mL. The expression of Aiolos (CST, Cat. # 15103) and the internal reference β-actin (CST, Cat. 5175S) were detected using a fully automated western blot quantitative analyser (Proteinsimple). The expression level of Aiolos relative to the internal reference was calculated using compass software. The remaining Aiolos protein relative to the vehicle control group at different doses was calculated using formula (5), and the Aiolos protein degradation relative to the vehicle control group at different doses was calculated using formula (6) to obtain the maximum degradation rate (Dmax) of Aiolos protein by the drug.

$$\text{Remaining Aiolos\%} = \text{Aiolos}_{\text{compound}} / \text{Aiolos}_{\text{vehicle}} \times 100\% \quad \text{formula (5)}$$

$$\text{Aiolos degradation\%} = 100\%\text{-formula (5)} \qquad \text{formula (6)}$$

Conclusion: The compounds of the present invention, such as the example compounds, have no obvious degradation effect on Aiolos protein in hPBMC cells.

**Test example 12: Study on IRAK4 degradation activity in hPBMC cells (4 hours)**

hPBMC cells are human peripheral blood mononuclear cells. Peripheral venous blood was taken from healthy volunteers and hPBMCs were isolated using Ficoll density gradient centrifugation (Ficoll-PaqueTM PLUS 1.077, GE, Cat. 17-1140-02). Culture conditions: RPMI-1640 + 10% FBS + 1% penicillin-streptomycin solution. The cells were cultured in an incubator at 37°C and 5% $CO_2$. The cells were plated in a 24-well plate at 1 × $10^6$ cells/well. After plating, compounds at different concentrations were added, and the cells were cultured in an incubator at 37°C and 5% $CO_2$ for 4 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, and the protein was quantified using a BCA kit (Beyotime, Cat. P0009), and then the protein was diluted to 1 mg/mL. The expression of IRAK4 (CST, Cat. 4363S) and the internal reference cofilin (CST, Cat. 5175S) were detected using a fully automated western blot quantitative analyser (Proteinsimple). The expression level of IRAK4 relative to the internal reference was calculated using compass software. The remaining IRAK4 protein relative to the vehicle control group at different doses was calculated using formula (7), and the IRAK4 protein degradation relative to the vehicle control group at different doses was calculated using formula (8), where $IRAK4_{compound}$ was the expression level of IRAK4 in the administration groups at different doses, and $IRAK4_{vehicle}$ was the expression level of IRAK4 in the vehicle control group. An IRAK4 degradation-drug concentration curve was fit using Graphpad Prism 8 software, and the $DC_{50}$ was calculated.

$$\text{Remaining IRAK4\%} = \text{IRAK4}_{\text{compound}} / \text{IRAK4}_{\text{vehicle}} \times 100\% \quad \text{formula (7)}$$

$$\text{IRAK4 degradation\%} = 100\%\text{-formula (7)} \qquad \text{formula (8)}$$

Conclusion: The compounds of the present invention, such as the example compounds, have a given degradation effect on IRAK4 protein in hPBMC cells at 4 h.

**Test example 13: Bioavailability test in beagle dogs**

After a single oral administration of the formulation samples prepared in foregoing examples of the present invention to male Beagle dogs (3 for each formulation), venous blood was taken at 0, 0.25, 0.5, 1, 2, 4, 6, 8, 10, 12, 24, 28 and 48 h, and the bioavailability was calculated from the area under the curve obtained from the points where the plasma concentration of compound 20 was taken as a function of time.

| | Example 14 | Example 15 |
|---|---|---|
| Cmax (ng/mL) | 63.3 | 51.6 |

(continued)

| | Example 14 | Example 15 |
|---|---|---|
| AUC (h·ng·mL-1) | 477 | 344 |
| F% | 5.62 | 4.03 |

## Claims

1. A pharmaceutical composition, **characterised in that** the pharmaceutical composition comprises an active ingredient A and a pharmaceutically acceptable excipient, and the active ingredient A is selected from a compound as represented by general formula (I) or a stereoisomer, a tautomer, a deuterated substance, a solvate, a prodrug, a metabolite, a pharmaceutically acceptable salt or an eutectic crystal thereof,

B-L-K (I);

wherein L is selected from -Ak1-Cy1-Ak2-Cy2-Ak3-Cy3-Ak4-Cy4-Ak5-;
Ak1, Ak2, Ak3, Ak4 and Ak5 are each independently selected from $-(CH_2)_q-$, O, $-(CH_2)_qNR^L-$, $NR^LC=O$, $C=ONR^L$, C=O, $-R^LC=CR^L-$, C=C or a bond;
$R^L$ is selected from H or $C_{1-6}$ alkyl;
Cy1, Cy2, Cy3 and Cy4 are each independently selected from a bond, 4- to 7-membered mono-heterocyclic ring, 4- to 10-membered fused-heterocyclic ring, 5-to 12-membered spiro-heterocyclic ring, 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S, or N;
B is selected from

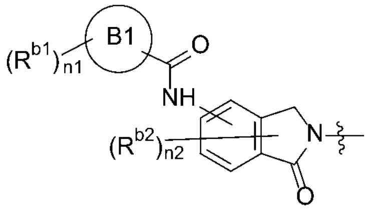 or 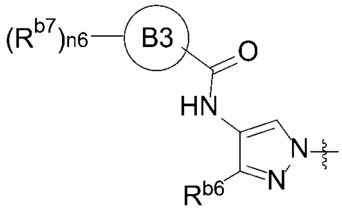 ;

B1 and B3 are each independently selected from $C_{6-10}$ aryl, 5- to 10-membered heteroaryl or 4- to 10-membered heterocyclyl, wherein the heteroaryl or heterocyclyl contains 1 to 4 heteroatoms selected from O, S or N;
$R^{b1}$ and $R^{b7}$ are each independently selected from H, F, Cl, Br, I, =O, OH, $NH_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $-N(R^{b21})_2$, $C_{6-10}$ aryl, 5- to 10-membered heteroaryl or 4- to 10-membered heterocyclyl, wherein the alkyl, alkoxy, cycloalkyl, heterocyclyl, aryl or heteroaryl is optionally further substituted with 0 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl, 5- to 10-membered heteroaryl, 4- to 10-membered heterocyclyl or $R^{b7a}$, and the heteroaryl or heterocyclyl contains 1 to 4 heteroatoms selected from O, S, or N;
$R^{b7a}$ is selected from $C_{1-4}$ alkyl, $-C_{3-6}$ cycloalkyl, 4- to 10-membered heterocyclyl, $-C_{1-4}$ alkylene $-C_{3-6}$ cycloalkyl, $-C_{1-4}$ alkylene 4- to 10-membered heterocyclyl, $-O-C_{3-6}$ cycloalkyl, -O-4- to 10-membered heterocyclyl, $-NH-C_{3-6}$ cycloalkyl, -NH-4- to 10-membered heterocyclyl, $-N(C_{1-4}$ alkyl$)-C_{3-6}$ cycloalkyl or $-N(C_{1-4}$ alkyl)-4- to 10-membered heterocyclyl, wherein the $R^{b7a}$ is optionally substituted with 1 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl or 4- to 10-membered heterocyclyl, and the heterocyclyl contains 1 to 4 heteroatoms selected from O, S, or N;
$R^{b2}$ and $R^{b6}$ are each independently selected from H, F, Cl, Br, I, =O, OH, $-C(=O)N(R^{b21})_2$, $-N(R^{b21})_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $C_{6-10}$ aryl, 5- to 10-membered

heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{1-4}$ alkoxy, $C_{3-6}$ cycloalkyl, 5- to 10-membered heteroaryl or 4- to 10-membered heterocyclyl, and the heteroaryl or heterocyclyl contains 1 to 4 heteroatoms selected from O, S, or N;

each $R^{b21}$ is independently selected from H, $C_{1-6}$ alkyl, $C_{1-4}$ alkoxy, $C_{3-6}$ cycloalkyl, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{3-6}$ cycloalkyl or $C_{1-4}$ alkoxy, and the heteroaryl or heterocyclyl contains 1 to 4 heteroatoms selected from O, S, or N;

n is selected from 0, 1, 2, 3 or 4;

K is selected from

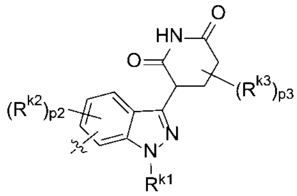 , 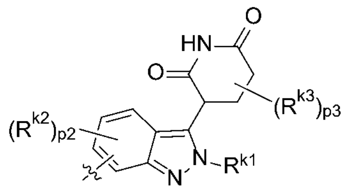 ,

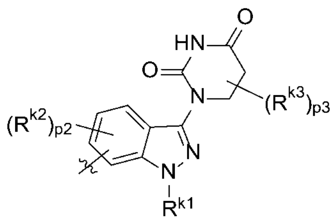 or 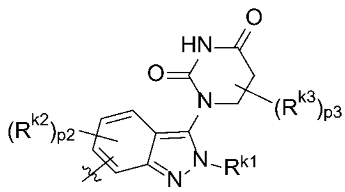 ;

each $R^{k1}$ is independently selected from H, $C_{1-4}$ alkyl, $C_{2-6}$ alkenyl, $C_{2-6}$ alkynyl, $C_{3-6}$ cycloalkyl or 3- to 6-membered heterocycloalkyl, wherein the alkyl, cycloalkyl or heterocycloalkyl is optionally further substituted with 0 to 4 substituents selected from H, F, Cl, Br, I, OH, $NH_2$, CN, $CF_3$, $C_{1-6}$ alkyl, $C_{1-6}$ alkoxy, $C_{2-6}$ alkenyl, $C_{2-6}$ alkynyl or $C_{3-6}$ cycloalkyl;

$R^{k2}$ and $R^{k3}$ are each independently selected from H, F, Cl, Br, I, OH, =O, $NH_2$, $CF_3$, CN, C(=O)OH, C(=O)$NH_2$, $C_{1-4}$ alkyl or $C_{1-4}$ alkoxy, wherein the alkyl or alkoxy is optionally further substituted with 0 to 4 substituents selected from H, F, Cl, Br, I, OH, or $NH_2$;

or two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, C(=O)OH, C(=O)$NH_2$, $C_{1-4}$ alkyl or $C_{1-4}$ alkoxy, and the heterocycle contains 1 to 4 heteroatoms selected from O, S or N;

q is selected from 0, 1, 2, 3 or 4;

n1, n2 and n6 are each independently selected from 0, 1, 2 or 3;

p2 and p3 are each independently selected from 0, 1, 2, 3 or 4;

optionally, 0 to 50 H of the compound as represented by general formula (I) are replaced by 0 to 50 D;

the pharmaceutical composition comprises 0.5-800 mg of the active ingredient A;

the content of the active ingredient A is selected from 0.5%-99.0%.

2. The pharmaceutical composition according to claim 1, **characterised in that** the active ingredient A is selected from a compound as represented by general formula (II) or a stereoisomer, a tautomer, a deuterated substance, a solvate, a prodrug, a metabolite, a pharmaceutically acceptable salt or an eutectic crystal thereof,

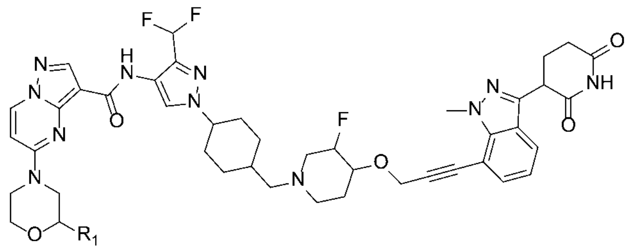

(II);

wherein $R_1$ is selected from $CF_3$, $CHF_2$, $CH_2CF_3$, $CH_2OH$, $CH_2OMe$, $CH_2CN$, or $CH_2$-cyclopropyl.

3. The pharmaceutical composition according to claim 1 or 2, **characterised by** comprising 1-600 mg of the active ingredient A, preferably 1-400 mg of the active ingredient A, further preferably 5-350 mg of the active ingredient A, and further preferably 10-200 mg of the active ingredient A.

4. The pharmaceutical composition according to claim 3, **characterised by** comprising 5 mg, 10 mg, 20 mg, 25 mg, 30 mg, 50 mg, 75 mg, 100 mg, 200 mg, 300 mg, 400 mg, 500 mg, or 600 mg of the active ingredient A.

5. The pharmaceutical composition according to claim 1, **characterised in that** the pharmaceutical composition comprises the active ingredient A and a pharmaceutically acceptable excipient, the active ingredient A is selected from the compound as represented by general formula (I) or a stereoisomer, a tautomer, a deuterated substance, a solvate, a prodrug, a metabolite, a pharmaceutically acceptable salt or an eutectic crystal thereof, and the structure of the compound as represented by general formula (I) is selected from one of the structures shown in Table S-1.

6. The pharmaceutical composition according to claim 1, **characterised in that** the structure of the compound as represented by general formula (I) is selected from the following structures:

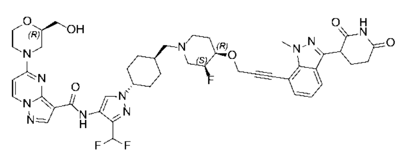

,

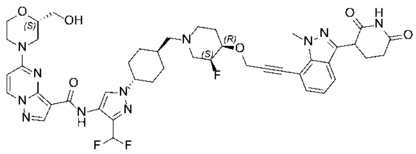

,

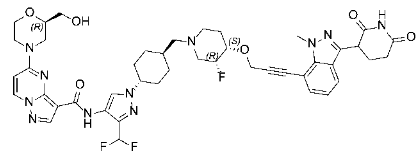

, and

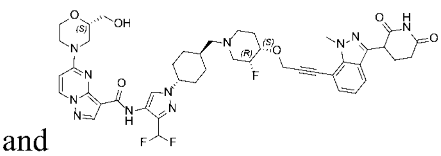

.

7. The pharmaceutical composition according to claim 1, **characterised in that** the pharmaceutically acceptable excipient contains one or both of a solubilizer or a solid dispersion carrier.

8. The pharmaceutical composition according to claim 7, **characterised in that** the solid dispersion carrier is selected from polymer carriers, preferably one or more of hydroxypropyl methylcellulose derivatives, ethyl celluloses, methyl celluloses, hydroxypropyl methylcellulose acetate succinate, povidones, copovidone, polyethylene glycols with an average molecular weight of not less than 1000, hydroxypropyl cellulose, polyvinyl caprolactam-polyvinyl acetate-polyethylene glycol graft polymers, polyacrylic resin, hydroxypropyl methylcellulose phthalate, or polyvinyl alcohol, and more preferably one or more of copovidone, hydroxypropyl cellulose, hydroxypropyl methylcellulose, Eudragit, hydroxypropyl methylcellulose acetate succinate (HPMCAS), or hydroxypropyl methylcellulose phthalate (HPMCP); the solid dispersion carrier is further preferably Eudragit, hydroxypropyl methylcellulose phthalate (HPMCP) or hydroxypropyl methylcellulose acetate succinate (HPMCAS), the weight ratio of the active ingredient A to the solid dispersion carrier is 1 : 0.01-1 : 100, preferably 1 : 0.5-1 : 10, and further preferably 1 : 1-1 : 6; optionally, the content of the solid dispersion in the pharmaceutical composition is 10%-100%, preferably 15%-65%, and further preferably 20%-60%.

9. The pharmaceutical composition according to claim 7, **characterised in that** the solubilizer is selected from one or

more of anionic surfactant, cationic surfactant, zwitterionic surfactant, nonionic surfactant, polymeric surfactant or cyclodextrin, the solubilizer is preferably sodium lauryl sulphate (SLS), vitamin E polyethylene glycol succinate (TPGS), polyethylene glycol and a derivative thereof, cyclodextrin or polyoxyethylene hydrogenated castor oil, and the weight ratio of the active ingredient A to the solubilizer is 1 : 0.01-1 : 100, preferably 1 : 0.05 -1 : 30, and further preferably 1 : 0.1-1 : 20.

**10.** The pharmaceutical composition according to any one of claims 8-9, **characterised by** further comprising a pharmaceutically acceptable excipient.

**11.** The pharmaceutical composition according to claim 10, **characterised in that** the pharmaceutically acceptable excipient further comprises one or more of a filler, a disintegrant, a binder, a suspending agent, an antioxidant, a colorant, a flavouring agent, a glidant, and a lubricant.

**12.** The pharmaceutical composition according to claim 11, **characterised in that**:

the filler is selected from one or more of lactose, microcrystalline cellulose, sucrose, glucose, powdered cellulose, calcium phosphate, dicalcium phosphate, calcium carbonate, aluminium silicate, dextrin, starch (including corn starch, potato starch or amylopectin), pregelatinized starch, sodium chloride, potassium chloride, mannitol or sorbitol, preferably one or more of lactose, microcrystalline cellulose, dicalcium phosphate, pregelatinized starch, or mannitol, and more preferably one or more of lactose, microcrystalline cellulose, dicalcium phosphate, or mannitol;
the disintegrant is selected from one or more of starch, pregelatinized starch, carboxymethyl starch sodium, crospovidone, and croscarmellose sodium, preferably one or more of carboxymethyl starch sodium, crospovidone, and croscarmellose sodium, and more preferably crospovidone and croscarmellose sodium;
the binder is selected from one or more of starch slurry, povidone, hydroxypropyl cellulose, hydroxypropyl methylcellulose, ethyl cellulose, sodium carboxymethyl cellulose, xanthan gum, arabic gum, gelatine, guar gum or carbomer, preferably one or more of povidone, hydroxypropyl cellulose, or hydroxypropyl methylcellulose, and more preferably one or more of povidone, hydroxypropyl methylcellulose E3, or hydroxypropyl methylcellulose E5;
the suspending agent is selected from one or more of arabic gum, tragacanth gum, xanthan gum, peach gum, sodium alginate, agar, starch slurry, methyl cellulose, sodium carboxymethyl cellulose, hydroxypropyl cellulose, carbomer, povidone, dextran, aluminium monostearate, or aluminium silicate, preferably one or more of xanthan gum, sodium alginate, methyl cellulose or hydroxypropyl cellulose, more preferably xanthan gum or hydroxypropyl cellulose;
the antioxidant is selected from one or more of butylated hydroxyanisole, dibutyl hydroxy toluene, propyl gallate, anhydrous sodium sulphite, sodium pyrosulphite, sodium thiosulphate, chelating agent, $\alpha$-tocopherol, thioglycerol, or sodium hydrogen sulphite, preferably one or more of butylated hydroxyanisole, dibutyl hydroxy toluene, $\alpha$-tocopherol, or sodium thiosulphate, and more preferably one or more of butylated hydroxyanisole, dibutyl hydroxy toluene, or $\alpha$-tocopherol;
the colorant is selected from one or more of iron oxide yellow, iron oxide red, pigments, and lakes, preferably one or more of iron oxide yellow, iron oxide red, lemon yellow, carminum, and blue aluminium lake, and more preferably iron oxide yellow and iron oxide red;
the flavouring agent is selected from one or more of stevioside, meringue, xylitol, high fructose, sodium cyclohexyl sulphamate, aspartame, neotame or sucralose, preferably aspartame or sucralose;
the glidant is selected from one or more of talc, colloidal silicon dioxide, polyethylene glycol, or magnesium lauryl sulphate, preferably colloidal silicon dioxide;
and the lubricant is selected from one or more of stearic acid, magnesium stearate, or sodium stearyl fumarate, preferably magnesium stearate or sodium stearyl fumarate.

**13.** The pharmaceutical composition according to claim 1, **characterised in that** the pharmaceutical formulation form prepared with the pharmaceutical composition is selected from a tablet, a granule, a capsule, a dry suspension, a powder, and an oral solution.

**14.** Use of the pharmaceutical composition according to any one of claims 1-13 in the preparation of a relevant drug for treating tumours or autoimmune system diseases.

## INTERNATIONAL SEARCH REPORT

International application No.
**PCT/CN2024/103473**

### A. CLASSIFICATION OF SUBJECT MATTER

A61K47/64(2017.01)i; A61K31/519(2006.01)i; A61K9/20(2006.01)i; A61K9/08(2006.01)i; A61K9/14(2006.01)i; A61P35/00(2006.01)i; A61P37/06(2006.01)i; A61P37/02(2006.01)i

According to International Patent Classification (IPC) or to both national classification and IPC

### B. FIELDS SEARCHED

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNABS; CNTXT; DWPI; ENTXT; ENTXTC; CNKI; STNext REGISTRY/CAPLUS: 降解; 基于式 (II) 和权利要求6中化合物的结构检索, structural search conducted based on formula (II) and compounds in claim 6; degradation; protac; IRAK4

### C. DOCUMENTS CONSIDERED TO BE RELEVANT

| Category* | Citation of document, with indication, where appropriate, of the relevant passages | Relevant to claim No. |
|---|---|---|
| PX | WO 2023131167 A1 (HAISCO PHARMACEUTICAL GROUP CO., LTD.; description, paragraph 183) 13 July 2023 (2023-07-13)<br>claims 1-14 | 1-14 |
| X | WO 2022161414 A1 (INCRELAND) 04 August 2022 (2022-08-04)<br>claims 1-8, 19 and 21, and description, paragraphs 0601, 0609 and 1868 | 1-14 |
| X | WO 2023036175 A1 (MEDSHINE DISCOVERY INC.) 16 March 2023 (2023-03-16)<br>claims 1-19 | 1-14 |
| A | CN 114437035 A (HAISCO PHARMACEUTICAL GROUP CO., LTD.) 06 May 2022 (2022-05-06)<br>entire document | 1-14 |
| A | CN 111655257 A (THE REGENTS OF THE UNIVERSITY OF MICHIGAN) 11 September 2020 (2020-09-11)<br>description, paragraphs 0305-0348 | 1-14 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 October 2024** | **10 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**
**Information on patent family members**

International application No.
**PCT/CN2024/103473**

| Patent document cited in search report | | | Publication date (day/month/year) | Patent family member(s) | | | Publication date (day/month/year) |
|---|---|---|---|---|---|---|---|
| WO | 2023131167 | A1 | 13 July 2023 | TW | 202333670 | A | 01 September 2023 |
| | | | | AU | 2023205573 | A1 | 15 August 2024 |
| WO | 2022161414 | A1 | 04 August 2022 | None | | | |
| WO | 2023036175 | A1 | 16 March 2023 | TW | 202317097 | A | 01 May 2023 |
| | | | | EP | 4400499 | A1 | 17 July 2024 |
| CN | 114437035 | A | 06 May 2022 | None | | | |
| CN | 111655257 | A | 11 September 2020 | JP | 2021502386 | A | 28 January 2021 |
| | | | | JP | 7424637 | B2 | 30 January 2024 |
| | | | | JP | 2024041896 | A | 27 March 2024 |
| | | | | EP | 3706736 | A1 | 16 September 2020 |
| | | | | EP | 3706736 | A4 | 11 August 2021 |
| | | | | US | 2020246474 | A1 | 06 August 2020 |
| | | | | US | 11147885 | B2 | 19 October 2021 |
| | | | | US | 2022072142 | A1 | 10 March 2022 |
| | | | | US | 11786602 | B2 | 17 October 2023 |
| | | | | US | 2022288217 | A1 | 15 September 2022 |
| | | | | US | 11833210 | B2 | 05 December 2023 |
| | | | | US | 2019142961 | A1 | 16 May 2019 |
| | | | | US | 11110177 | B2 | 07 September 2021 |
| | | | | US | 2024277856 | A1 | 22 August 2024 |
| | | | | US | 2019144442 | A1 | 16 May 2019 |
| | | | | US | 10632209 | B2 | 28 April 2020 |
| | | | | CA | 3082086 | A1 | 16 May 2019 |
| | | | | EP | 3706737 | A1 | 16 September 2020 |
| | | | | EP | 3706737 | A4 | 20 October 2021 |
| | | | | JP | 2021502388 | A | 28 January 2021 |
| | | | | WO | 2019094773 | A1 | 16 May 2019 |
| | | | | WO | 2019094772 | A1 | 16 May 2019 |
| | | | | CA | 3082077 | A1 | 16 May 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023070367 W
- WO 2021158634 A
- WO 2020113233 A1